# EUROPEAN PATENT SPECIFICATION

(11) **EP 2 659 404 B1**
(45) Date of publication and mention of the grant of the patent: **08.08.2018**
(21) Application number: 11854432.9
(22) Date of filing: 28.12.2011
(51) Int. Cl.: C12N 5/071, G01N 33/50

(54) **CELLS HAVING DISRUPTED EXPRESSION OF PROTEINS INVOLVED IN ADME AND TOXICOLOGY PROCESSES**
ZELLEN MIT GESTÖRTER EXPRESSION VON IN ADME UND TOXIKOLOGIEVERFAHREN INVOLVIERTEN PROTEINEN
CELLULES DONT L'EXPRESSION DES PROTÉINES IMPLIQUÉES DANS LES PROCESSUS ADME ET TOXICOLOGIQUES EST PERTURBÉE

(30) Priority: 29.12.2010 US 201061427969 P; 15.03.2011 US 201161452842 P; 15.03.2011 US 201161452846 P
(43) Date of publication of application: 06.11.2013
(60) Divisional application: 18181040.9
(73) Proprietor: Sigma-Aldrich Co., LLC, St. Louis, MO 63103 (US)
(72) Inventor: BOURNER, Maureen, St. Louis Missouri 63103 (US); BRAYMAN, Timothy, St. Louis Missouri 63103 (US); DAVIS, Gary, St. Louis Missouri 63103 (US); MITCHELL, Michael D., St. Louis Missouri 63103 (US); THOMPSON, David C., St. Louis Missouri 63103 (US); XIAO, Yongling, St. Louis Missouri 63103 (US)
(74) Representative: Murgitroyd & Company
(86) International application number: PCT/US2011/067608
(87) International publication number: WO 2012/092379

(56) References cited:
- WO-A1-01/79845
- WO-A2-2008/060489
- WO-A2-2009/049284
- WO-A2-2012/082509
- JP-A- 2005 229 967
- US-A1- 2003 083 485
- US-A1- 2005 106 635
- US-A1- 2009 137 517
- US-A1- 2010 184 742
- US-A1- 2010 218 264
- US-A1- 2010 240 090
- US-A1- 2010 323 371
- GLAVINAS ET AL.: 'The Role of ABC Transporters in Drug Resistance, Metabolism and Toxicity' CURRENT DRUG DELIVERY vol. 1, no. 1, 2004, pages 27 - 42, XP008120176

## Description

### FIELD OF THE INVENTION

The present disclosure generally relates to cells having disrupted expression of ABCG2 encoded protein involved in a drug absorption, distribution, metabolism and excretion (ADME) and/or toxicology process.

### BACKGROUND OF THE INVENTION

The pharmacokinetic, efficacy and safety profiles of new drug candidates may be significantly affected by interactions with membrane transporters, drug metabolizing enzymes (DMEs), and cell stress response pathways. For example, interactions with membrane transporters influence the intestinal absorption, systemic distribution and renal clearance of a drug, while drug metabolism pathways play a major role in the pharmacokinetic profile, metabolic clearance of the parent molecule and formation of potentially harmful metabolites. These two systems (transporters and drug metabolism pathways) are also key determinants of assessing the potential for harmful drug-drug interactions (DDis). Xenobiotic sensors and cell stress response pathways enable cells to react to exposure to a foreign substance and often mediate toxic and adverse side effects of the compound. Therefore, a better understanding of the potential interactions and adverse effects of new drug candidates on these systems is key to improving the overall early drug discovery screening process. In addition, requests for additional information on these systems from regulatory agencies appear to be increasing.

There are numerous members of the membrane transporter, drug metabolism, xenobiotic sensor and stress response pathways. For many of these, the specific function, substrates, and/or inhibitors have yet to be described. For example, there are more than 400 transporters and at least 57 cytochrome p450 (drug metabolism) genes known in the human genome. The more known about each of the above described systems, the more accurate the preclinical drug screening process could become. The vast majority of drugs (approximately 91%) fail to successfully complete the three phases of drug testing in humans. A majority of those drugs that fail, do so because of unforeseen toxicology in human patients, despite the fact that all of these drugs had been tested in animal models and were found to be safe. This is due, in part, to the fact that toxicology testing is performed in animals, and animal proteins differ from the orthologous proteins in humans, and also to the lack of appropriate test systems using human cells.

US 2010/0184742 discusses prognosis of a clinical response in a human patient to a medicament which acts on the central nervous system and which is a substrate of the ABCB1 protein and the treatment of human patients having specific polymorphisms in the ABCB1 gene.

US 2010/0240090 discusses methods for identifying an agent that connects a phenotype associated with a health condition or predisposition for a health condition.

US 2003/0083485 discusses a novel P450 2D6 gene variant and methods for detecting drug sensitivity.

US 2005/0106635 discusses methods for identifying agents which alter thyroid hormone metabolism or cholesterol and lipid metabolism by modulating expression and/or activity of a nuclear receptor CAR.

US 2009/0137817 discusses a method of sensitizing a cell to cancer treatment.

US 2010/0323371 discusses knockout mice for the Ca²⁺ sensor membrane protein STIM-1, STIM-2, or both, as well as cell lines from these knock out mice.

Current Drug Delivery, (2004) vol 1, no. 1 pages 27-42, Glavinas et al: "The Role of ABC Transporters in Drug Resistance, Metabolism and Toxicity" discusses ATP Binding Cassette (ABC) transporters and their role in Absorption-Distribution-Metabolism-Excretion (ADME) and toxicology, discusses human ABC transporters, their expression, localization and basic mechanism of action.

US 2010/0218264 discusses methods and compositions for genome editing of one or more loci in a rat, using fusion proteins comprising a zinc-finger protein and a cleavage domain or cleavage half domain.

WO 2009/049284 discusses cell lines comprising disrupted sialidase expression and methods of using the cell lines to produce glycoproteins with enhanced sialylation.

WO 2008/060489 discusses nucleic acids and vectors for interfering with the expression of membrane efflux transport proteins in cells, cell lines including such nucleic acids and methods for screening chemicals and biomolecules for gastrointestinal adsorption in animals.

JP 2005 229967 discusses recombinant human cells for evaluating drug metabolism.

WO 01/79845 discusses identification of compounds that alter protein expression and the evaluation of compounds for their ability to induce expression of a gene encoding a protein involved in drug metabolism.

Therefore, what is needed in the art are human cells that are mutated for the endogenous genes involved in ADME and toxicology processes, such as transporters, DMEs, xenobiotic sensors, and cell stress response pathways, whether the mutation leads to disrupted expression as a knockout, a knock-down, a knock-in, overexpression, or clinically significant variants with SNPs, or any combination thereof. Such genetically modified cells would provide powerful new tools that may be used to improve the preclinical drug discovery process and identify new compounds that are both safe and efficacious.

### SUMMARY OF THE INVENTION

Briefly, therefore, one aspect of the present disclosure provides a human cell line comprising disrupted expression of at least one protein chosen from a membrane transporter, a drug metabolizing enzyme, a xenobiotic sensor, a cellular stress response pathway protein, and combinations thereof.

Another aspect of the disclosure encompasses a method for assessing the effect of an agent, wherein the method contacting a cell with disrupted expression of at least one transporter, drug metabolism enzyme, xenobiotic sensor or cell stress response pathway with the agent, and comparing results of a selected parameter to results obtained from contacting a comparable wild-type cell with the same agent.

The present invention provides a genetically modified human cell line comprising disrupted expression of an endogenous genomic sequence encoding a protein involved in a drug absorption, distribution, metabolism and excretion and/or toxicology process, wherein the endogenous genomic sequence is ABCG2 and wherein expression is disrupted by targeting endonuclease mediated genome editing.

The invention further provides a method for assessing the effect of an agent, the method comprising:
a) contacting a human cell line comprising disrupted expression of an endogenous genomic sequence ABCG2 wherein expression is disrupted by targeting endonuclease mediated genome editing with the agent; and
b) comparing results of a selected parameter to results obtained from contacting a comparable wild-type cell with the same agent.

Other aspects and iterations of the disclosure are described in more detail below.

### DESCRIPTION OF THE FIGURES

FIG. **1** illustrates the efflux ratios of three compounds (estrone-3-sulfate, digoxin and CDCFDA) in the wildtype (WT) (open bars) intestinal epithelial cell line C2BBe1 versus the same line with the ABCG2 (BCRP) transporter functional knockout (BCRP/KO) (solid bars). Atenolol and metoprolol were used as control compounds.
FIG. **2** compares the efflux ratios of estrone-3-sulfate and digoxin in two single transporter knockout cell lines (BCRP KO and MDR1 KO) and a double knockout cell line (MDR1/BCRP KO) versus the parental cell line (C2BBe1, WT). Metoprolol was used as a control compound.

### DETAILED DESCRIPTION OF THE INVENTION

Disclosed herein is the provision of populations of cells comprising disrupted expression of at least one ADME/Tox protein chosen from membrane transporters, drug metabolizing enzymes, xenobiotic sensors, and cellular stress response pathway proteins. The cells of the invention may comprise inactivated chromosomal sequence(s) such that expression of at least one ADME/Tox protein is substantially eliminated. Also provided are methods of using the cells of the invention to screen agents or to assess the effects of agent in the cells comprising disrupted expression of the ADME/Tox protein or proteins.

### (l) Cells Comprising Disrupted Expression of ADME/Tox Protein(s)

One aspect of the present disclosure encompasses a cell comprising disrupted expression of a protein involved in a drug absorption, distribution, metabolism, and excretion (ADME) and/or toxicology (Tox) process according to claim 1. As used herein, the term "ADME/Tox" refers to proteins (and their corresponding genes) that are involved drug absorption, distribution, metabolism, excretion, and/or toxicology
processes. ADME/Tox proteins include, but are not limited to, membrane transporters, drug metabolizing enzymes, xenobiotic sensors, and cell stress response proteins. Because of the disrupted expression of at least one ADME/Tox protein, the cells disclosed herein may have altered responses with regard to drug absorption, disposition, and/or response.

In the cell disclosed herein, expression of the at least one ADME/Tox protein is disrupted by targeting endonuclease mediated genome editing of the endogenous genomic sequence ABCG2 such that the cell produces substantially no functional protein (i.e., the cell may be termed a knockout), a reduced level of the protein (i.e., the cell may be termed a knock-down), an increased level of the protein, or a modified version of the protein.

The disrupted expression of the ADME/Tox protein may be due to modification of a chromosomal sequence ABCG2 encoding the ADME/Tox protein. The cell may be homozygous, heterozygous, or hemizygous for the modified chromosomal sequence.

The chromosomal sequence encoding the ADME/Tox protein may be modified by a deletion of at least one nucleotide, an insertion of at least one nucleotide, or a substitution of at least one nucleotide. In some embodiments, the chromosomal sequence ABCG2 encoding the ADME/Tox protein may be inactivated by at least one deletion such that substantially no protein is made by the cell. For example, the chromosomal sequence ABCG2 encoding the ADME/Tox protein may comprise a deletion of all or part of the coding region, a deletion of all or part of a control region, a deletion or disruption of a splice site, a substitution that introduces a premature codon, or combinations thereof such that the cell produces essentially no functional protein.

In other embodiments, the chromosomal sequence ABCG2 encoding the ADME/Tox protein may be modified such that reduced levels of the protein are made. For example, the chromosomal sequence ABCG2 encoding the ADME/Tox protein may comprise a deletion, insertion, and/or substitution in the coding region and/or a deletion, insertion, and/or substitution in a control region such that the cell produces the protein at reduced levels.

In still other embodiments, the chromosomal sequence ABCG2 encoding the ADME/Tox protein may be modified such that an altered version of the protein is produced. For example, chromosomal sequence may be modified to comprise a single nucleotide change (e.g., a SNP). Depending upon the location of the SNP, an altered version of the protein may be produced by the cell. Alternatively, the chromosomal sequence encoding the ADME/Tox protein may be modified by a deletion, insertion, or substitution in the coding region such that the cell produces an altered version of the protein. The altered protein may have altered substrate specificity, altered binding interactions, altered kinetics, altered transport rates, altered directionality, altered cellular localization, altered protein interactions, altered stability, and so forth.

The chromosomal sequence ABCG2 encoding the ADME/Tox protein may be modified via a deletion, insertion, and/or substitution such that the cell produces the protein at increased levels. That is, the protein may be overexpressed.

The cell comprises disrupted expression of ABCG2 encoded ADME/Tox protein, and in another embodiment, the cell may comprise disrupted expression of another ADME/Tox protein, n still another embodiment, the cell may comprise disrupted expression of another two ADME/Tox proteins, in a further embodiment, the cell may comprise disrupted expression of another three ADME/Tox proteins, and in yet another embodiment, the cell may comprise disrupted expression of another four or more ADME/Tox proteins.

In another embodiment, the cell comprises one inactivated chromosomal sequence such that the expression of the ADME/Tox protein encoded by ABCG2 is substantially eliminated (i.e., knocked out), in still another embodiment, the cell may comprise another inactivated chromosomal sequence such that expression of two ADME/Tox proteins are substantially eliminated (i.e., knocked out),in an alternate embodiment, the cell may comprise another two inactivated chromosomal sequences such that expression of three ADME/Tox proteins are substantially eliminated (i.e., knocked out),in yet another embodiment, the cell may comprise another three or more inactivated
chromosomal sequences such that expression of four or more ADME/Tox proteins are substantially eliminated (i.e., knocked out).

In a further embodiment, the cell may comprise disrupted expression of membrane transporter encoded by ABCG2 and, optionally, disrupted expression of at least one additional ADME/Tox protein. Disclosed herein, the cell may comprise disrupted expression of at least one drug metabolizing enzyme and, optionally, disrupted expression of at least one additional ADME/Tox protein. Disclosed herein the cell may comprise disrupted expression of at least one xenobiotic sensor and, optionally, disrupted expression of at least one additional ADME/Tox protein. Disclosed herein the cell may comprise disrupted expression of at least one cellular stress response protein and, optionally, disrupted expression of at least one additional ADME/Tox protein.

Examples of membrane transporters, drug metabolizing enzymes, xenobiotic sensors, and cell stress response proteins are detailed below.

It should be understood that the gene designations as used herein, while referring to the human genes, encompass the close homologs of any of these that have been identified within other animals including invertebrates such as C. elegans and D. melanogaster, and mammals, including but not limited to mouse, rats, hamsters, cats, dogs and monkeys. Close homologs can be identified by sequence analyses, phylogenetic analyses, functional assays, and the like.

### (a) Transporter Proteins

An ADME/Tox protein may be a membrane transporter. Transporters are involved in the movement of molecules across cell membranes. The transporter proteins involved in drug absorption and disposition represent members from two major groups, i.e., the ATP-binding cassette (ABC) transporter superfamily and the solute carrier (SLC) superfamily. There are currently 7 subfamilies of ABC transporters with at least 49 genes known. Several members of the ABC transporter superfamily are known to be involved in drug resistance (e.g. BCRP,
MDR1). By comparison, the SLC transporters include 55 families with at least 362 functional protein-coding genes. The most relevant members of both the ABC and SLC transporter families with regard to ADME/Tox applications are listed below in Tables A and B.

| **TABLE A: Human ATP-Binding Cassette (ABC) Transporters** | | | | |
|---|---|---|---|---|
| **Sub-Family** | **Gene symbol** | **Gene common name** | **UniProtKB/ Swiss-Prot Access No.** | **Protein Annotation** |
| **ABCA** | ABCA2 | ABC2 | Q9BZC7 | ATP-binding cassette, sub-family A, member 2 |
| **ABCB** | ABCB1 | MDR1 | P08183 | Multi-drug resistance 1 (P-glycoprotein) |
| | ABCB4 | MDR3 | P21439 | Multi-drug resistance 3 |
| | ABCB11 | BSEP/ SPGP | O95342 | Bile salt export protein |
| **ABCC** | ABCC1 | MRP1 | P33527 | Multidrug resistance-associated protein 1 |
| | ABCC2 | MRP2 | Q92887 | Multidrug resistance-associated protein 2 |
| | ABCC3 | MRP3 | O15438 | Multidrug resistance-associated protein 3 |
| | ABCC4 | MRP4 | O15439 | Multidrug resistance-associated protein 4 |
| | ABCC5 | MRP5 | O15440 | Multidrug resistance-associated protein 5 |
| | ABCC6 | MRP6 | O95255 | Multidrug resistance-associated protein 6 |
| | ABCC7 | CFTR | P13569 | Cystic fibrosis transmembrane conductance regulator |
| | ABCC8 | SUR1 | Q09428 | Sulfonylurea receptor 1 |
| | ABCC9 | SUR2 | Q60706 | Sulfonylurea receptor 2 |
| | ABCC10 | MRP7 | Q5T3U5 | Multidrug resistance-associated protein 7 |
| | ABCC11 | MRP8 | Q96J66 | Multidrug resistance-associated protein 8 |
| | ABCC12 | MRP9 | Q96J65 | Multidrug resistance-associated protein 9 |
| | ABCC13 | ABCC13 | Q9NSE7 | ATP-binding cassette, sub-family C, member 13 |
| **ABDG** | ABCG2 | BCRP | Q9UNQ0 | Breast cancer resistance protein |

| **TABLE B. Human Solute Carrier (SLC) Transporters** | | | | |
|---|---|---|---|---|
| **Family** | **Gene symbol** | **Gene common name** | **UniProtKB/ Swiss-Prot Access No.** | **Protein Annotation** |
| **SLC10** | SLC10A1 | NTCP | Q14973 | Sodium taurocholate co-transporting polypeptide |
| | SLC10A2 | IBAT/ ASBT | Q12864 | Apical sodium-dependent bile acid transporter |
| **SLC15** | SLC15A1 | PEPT1 | P46059 | Oligopeptide transporter 1 |
| | SLC15A2 | PEPT2 | Q16348 | Oligopeptide transporter 2 |
| **SLC16** | SLC16A1 | MCT1 | P53985 | Monocarboxylate transporter 1 |
| | SLC16A7 | MCT2 | O60669 | Monocarboxylate transporter 2 |
| | SLC16A8 | MCT3 | O95907 | Monocarboxylate transporter 3 |
| | SLC16A3 | MCT4 | O15427 | Monocarboxylate transporter 4 |
| | SLC16A4 | MCT5 | O15374 | Monocarboxylate transporter 5 |
| | SLC16A5 | MCT6 | O15375 | Monocarboxylate transporter 6 |
| **SLC21/ SLCO** | SLCO1A2 | OATP1A2 | P46721 | Organic anion transporting polypeptide 1A2 |
| | SLCO1 B1 | OATP1B1 | Q9Y6L6 | Organic anion transporting polypeptide 1B1 |
| | SLCO1B3 | OATP1B3 | Q9NPD5 | Organic anion transporting polypeptide 1B3 |
| | SLCO1C1 | OATP-F | Q9NYB5 | Organic anion transporting polypeptide 1C1 |
| | SLCO2B1 | OATP-B | O94956 | Organic anion transporting polypeptide 2B1 |
| | SLCO3A1 | OATP3A1 | Q9UIG8 | Organic anion transporting polypeptide 3A1 |
| | SLCO4A1 | OATP-E | Q96BD0 | Organic anion transporting polypeptide 4A1 |
| | SLCO4C1 | OATP-H | Q6ZQN7 | Organic anion transporting polypeptide 4C1 |
| **SLC22** | SLC22A1 | OCT1 | 015245 | Organic cation transporter 1 |
| | SLC22A2 | OCT2 | 015244 | Organic cation transporter 2 |
| | SLC22A3 | OCT3 | 075751 | Organic cation transporter 3 |
| | SLC22A4 | OCTN1 | Q9H015 | Organic cation transporter, novel, type 1 |
| | SLC22A5 | OCTN2 | O76082 | Organic cation transporter, novel, type 2 |
| | SLC22A6 | OAT1 | Q4U2R8 | Organic anion transporter 1 |
| | SLC22A7 | OAT2 | Q9Y694 | Organic anion transporter 2 |
| | SLC22A8 | OAT3 | Q8TCC7 | Organic anion transporter 3 |
| | SLC22A9 | OAT7 | Q8IVM8 | Organic anion transporter 7 |
| | SLC22A11 | OAT4 | Q9NSA0 | Organic anion transporter 4 |
| **SLC47** | SLC47A1 | MATE1 | Q96FL8 | Multidrug and toxin extrusion protein 1 |
| | SLC47A2 | MATE2 | Q86VL8 | Multidrug and toxin extrusion protein 2 |
| **MISC** | n/a | OSTA | Q86UW1 | Organic solute transporter alpha |
| | n/a | OSTB | Q86UW2 | Organic solute transporter beta |

### (i) ABC transporter superfamily

ABC transporter proteins are a large and important superfamily of membrane transport proteins, ubiquitous in the animal kingdom. These transmembrane proteins hydrolyze ATP and use the energy to power various other functions, including translocation of molecules across intracellular and extracellular membranes, often against a concentration gradient. (For reviews, see Higgins, C. F., ABC transporters: from microorganisms to man, Annu. Rev. Cell Biol. 8 67-113 (1992); Dean M., Human ABC Transporter Superfamily, Bethesda (MD): National Center for Biotechnology Information (US); November 18, 2002, available online at www. ncbi.nlm.nih.gov/bookshelf/br.fcgi?book=mono_001; and Vasiliou V et al. Human ATP-binding cassette (ABC) transporter family, Hum Genomics 3, 281-290 (2009)). Non-limiting examples of human ABC transporter genes relevant to ADME/Tox applications are listed in Table A.

Genetic variation in ABC transporter genes may be causative or contributor to a wide variety of human disorders. For example, mutations in CFTR (cystic fibrosis transmembrane conductance regulator) protein are involved in cystic fibrosis. Additionally, overexpression of certain ABC transporters occurs in cancer cell lines or tumors that are multidrug resistant, apparently allowing certain cancer cells to extrude certain chemotherapeutic agents. Non-limiting examples ABC transporters linked to a disease or condition include, for example, ABCA1 linked to Tangier disease and familial hypoapoproteinemia; ABCA4 linked to Stargardt's disease, fundus flavimaculatis, retinitis pigmentosum, cone-rod dystrophy, and age-related macular degeneration; ABCB1 linked to ivermectin susceptibility and digoxin uptake; ABCB2 linked to immune deficiency; ABCB3 linked to immune deficiency; ABCB4 linked to progressive familial intrahepatic cholestasis and intrahepatic cholestasis of pregnancy; ABCB7 linked to X- linked sideroblastosis and anemia; ABCB11 linked to progressive familial intrahepatic cholestasis; ABCC2 linked to Dubin-Johnson Syndrome; ABCC6 linked to pseudoxanthoma elasticum; ABCC7 linked to cystic fibrosis, congential bilateral absence of the vas deferens, pancreatitis, and bronchiectasis; ABCC8 linked to familial persistent hyperinsulinemic hypoglycemia of infancy; ABCD1 linked to adrenoleukodystrophy; and ABCG5 linked to sitosterolemia.

The superfamily of ABC transporters is further subdivided into subfamilies, based on phylogenetic analysis and intron structure.

### 1) ABCA subfamily

This subfamily includes twelve full transporters, including a first subgroup of seven genes scattered across six different chromosomes (ABCA1, ABCA2, ABCA3, ABCA4, ABCA7, ABCA12, ABCA13) and a second subgroup of five genes (ABCA5, ABCA6, ABCA8, ABCA9, ABCA10) clustered together on chromosome 17q24. ABCA1 and ABCA4 (ABCR) have been studied in depth, revealing involvement of the ABCA1 protein in disorders of cholesterol transport and HDL biosynthesis, and of the ABCA4 protein in vision, because it transports vitamin A derivatives in rod photoreceptor outer segments. Exemplary members are detailed below.

*ABCA2:* The full length human *ABCA2* cDNA and its detailed expression pattern have been fully characterized. ABCA2 has shown the highest levels in fetal and adult brain, spinal cord, ovary, prostate and leukocytes. Expression of ABCA2 has been detected in other tissues, including lung, kidney, heart, liver, skeletal muscle, pancreas, testis, spleen, colon and fetal liver. Cellular immunolocalization of ABCA2 protein revealed a distinct, punctate staining corresponding to vesicles that were hypothesized to be peroxisomes. Further analysis using vesicle-specific antibodies revealed a colocalization of ABCA2 with late endolysomes and transgolgi organelles. ABCA2 protein shares the most homology with other A subfamily proteins, including ABCA1, ABCA3, ABCA, ABCA6, ABCA7. A lipocalin signature motif in the ABCA2 sequence suggests a function in the transport of lipids, steroids and structurally similar molecules, such as estramustine and estradiol.

### 2) ABCB (MDRITAP) subfamily

This subfamily includes four full transporters and seven half transporters. The ABCB2 and ABCB3 (TAP) genes are half transporters that form a heterodimer that transports peptides into the ER which are then presented as antigens by class I HLA molecules. The ABCB9 half transporter has been localized to lysosomes. ABCB6, ABCB7, ABCB8, and ABCB1 0, all half transporters, are located in the mitochondria, where they function in iron metabolism and transport of Fe/S protein precursors. Exemplary ABCB subfamily members are detailed below.

*ABCB1:* ABCA1 is also known as MDR1 or PGY1, encoding a transporter protein that functions at the blood-brain barrier and in the liver, and is known to confer an MDR phenotype to cancer cells. ABCB1 maps to chromosome 7q21.1 and is a well characterized ABC transporter, known to confer a multidrug resistance phenotype to cancer cells that developed resistance to chemotherapy drugs. The transporter moves hydrophobic substrates including drugs such as colchicine, etoposide (VP16), Adriamycin, and vinblastine, and also lipids, steroids, xenobiotics, and peptides. It is expressed in cells at the blood-brain barrier and thought to transport compounds into the brain that are not amenable to delivery by diffusion. The protein is also expressed in many secretory cell types such as kidney, liver, intestine, and the adrenal gland, where its normal function likely involves excreting toxic metabolites. ABCB1 is also highly expressed in hematopoietic stem cells and may protect cells against the effects of cytotoxins. ABCB1 is also implicated in the migration of dendritic cells. ABCB1 expression has been detected in intestine, liver and kidney.

*ABCB4:* Also known as MDR3, ABCB4 protein is expressed in the liver and involved in the secretion of bile acids. ABCA4 is expressed in intestine. The ABCB4 protein transports certain phospholipids across cell membranes. Large amounts of bile acids are potentially harmful to cells unless they are bound to phospholipids. Some ABCB4 gene mutations impair the movement of phospholipids across cell membranes, leading to a lack of phospholipids available to bind to bile acids. A buildup of free bile acids damage liver cells, which causes the signs and symptoms of liver disease. Therefore ABCA4 is believed to play an import role in liver cell function. Another group of mutations in the ABCB4 gene have been found to cause progressive familial intrahepatic cholestasis type 3 (PFIC3). Women with certain ABCB4 gene mutation are at risk of having intrahepatic cholestasis condition during pregnancy (ICP).

*ABCB11:* Also known as BSEP (bile salt export pump) or SPGP, ABCB11 is expressed in the liver and kidney. More than 100 mutations in the ABCB11 gene have been found to cause progressive familial intrahepatic cholestasis type 2 (PFIC2). People with no functional BSEP protein also seem to be at a greater risk of developing hepatocellular carcinoma. Mutations in the ABCB11 gene prevent the BSEP protein from effectively transporting bile salts out of the liver. This lack of transport causes bile salts to build up in liver cells, leading to liver disease and its associated signs and symptoms. Other disorders, such as benign recurrent intrahepatic cholestasis (BRIC), intrahepatic cholestasis of pregnancy (ICP), are also associated with the ABCB11 gene.

### 3) ABCC (CFTR/MRP) subfamily

The ABCC subfamily includes twelve full transporters, including the CFTR protein, which is a chloride ion channel that plays a role in all exocrine secretions. Exemplary members are detailed below.

*ABCC1:* ABCC1 encodes multidrug resistance-associated protein 1 (MRP1), which functions a multispecific anion transporter. The ABCC1 gene maps to chromosome 16p13.1 and is expressed in tumor cells. The ABCC1 (MRP1) pump confers resistance to doxorubicin, daunorubicin, vincristine, colchicines, and several other compounds, with effects comparable to those of ABCB1. However, unlike ABCB1, ABCC1 transports drugs that are conjugated to glutathione by the glutathione reductase pathway. Disruption of the Abcc1 gene in mice impairs their inflammatory response and imparts hypersensitivity to the anticancer drug etoposide. The ABCC1 protein is also believed to help protect cells from chemical toxicity and oxidative stress, and to mediate inflammatory responses involving cysteinyl leukotrienes. Evidence showed that ABCC1 is expressed in the intestine, liver and kidney.

*ABCC2:* Also known as MRP2, ABCC2 maps to chromosome 1Oq24 and is localized to canalicular cells in the liver. It is a major exporter of organic anions from the liver into the bile. The MRP2 gene is known to be mutated in the TR rat, a rat strain characterized by jaundice and a deficiency in organic ion transport. The gene is also mutated in human Dubin-Johnson syndrome patients, who suffer the symptoms of a disruption of organic ion transport. Evidence also implicates MRP2 overexpression in drug resistance. Expression of ABCC2 has been detected in intestine, liver and kidney.

*ABCC3:* Also known as MRP3, ABCC3 is expressed in intestine, liver and kidney. The protein encoded by ABCC3 is canalicular multispecific organic anion transporter 2. ABCC2 and ABCC3 mediate the elimination of toxic compounds, such as drugs and carcinogens, and have a large overlap in substrate specificity. For example, ABCC3 protein was shown to transport the anticancer drug methotrexate (MTX) and its toxic metabolite 7-hydroxymethotrexate from the liver into the circulation, leading to increased plasma levels and urinary excretion and thus has profound effects on the elimination and severity of toxicity of MTX. ABCC3 also protects against trabectedin-mediated hepatotoxicity. In addition, ABCC3 plays major roles in the efflux transport of various glucuronide conjugates from the enterocytes to the portal blood in the small intestine.

*ABCC4:* Also known as the multidrug resistance-associated protein 4 (MRP4) or multi-specific organic anion transporter B (MOAT-B), ABCC4 acts as a regulator of intracellular cyclic nucleotide levels and as a mediator of cAMP-dependent signal transduction to the nucleus. The functional importance of MRP4 in the urinary excretion of hydrochlorothiazide (HCT) and furosemide was investigated using gene knockout mice. The renal clearance of HCT and furosemide was reduced significantly but not abolished in Mrp4 knockout mice. Mrp4, therefore, together with other unknown transporters, accounts for the luminal efflux of HCT and furosemide from proximal tubular epithelial cells. In addition, the elimination of topotecan from the brain was delayed and that the concentration of topotecan in the cerebrospinal fluid was greatly enhanced in Mrp4 knockout mice, which indicates the role of ABCC4 in the uptake and efflux of topotecan across the brain-blood barrier.

*ABCC5:* ABCC5 (MRP5) is a typical organic anion pump but without an N-terminal transmembrane domain in comparison to other ABCC proteins. In vitro transport studies identified ABCC5 as a cellular export pump for numerous compounds including cGMP, nucleoside monophosphate analogs, heavy metal compounds and fluorochromes. ABCC5-transfected cells also exhibit resistance to anticancer and antiviral drugs. The ABCC5 gene is widely transcribed among human tissues with highest levels in heart, brain, skeletal muscle, kidney and testis. The various ABCC5 transcripts, the splice variants formed by the alternative usage of a cryptic donor splice site, are abundantly expressed in the human retina but are also present in many other tissues at varying levels. Alternative splicing of the ABCC5 mRNA may provide an elegant mechanism for tissue-dependant regulation of ABCC5 gene expression.

ABCC6: Also known as MRP6, ABCC6 is a membrane transporter whose deficiency leads to a connective tissue disorder. Specifically, pseudoxanthoma elasticum (PXE), a genetic disease characterized by calcification and fragmentation of elastic fibers of the skin, cardiovascular system and eye, is caused by mutations of the ABCC6 gene. The expression of (mouse) Abcc6 is highly correlated with the local mineralization regulatory system and the BMP2-Wnt signaling pathway known to be involved in the systemic regulation of calcification, suggesting potential pathways for the action of Abcc6 in myocardial calcification. ABCC6 is also involved in sterol transport, as sterols are preferential regulators of ABCA transporter activity and expression.

*ABCC7:* Also known as CFTR (transmembrane conductance regulator), ABCC7 functions as a chloride channel and controls the regulation of other transport pathways. ABCC7 may also regulate bicarbonate secretion and salvage in epithelial cells by regulating the SLC4A7 transporter. Mutations in this gene are associated with the autosomal recessive disorders cystic fibrosis and congenital bilateral aplasia of the vas deferens. More than a thousand different mutations in the CFTR gene have been detected worldwide. Alternatively spliced transcript variants have also been described, many of which result from mutations in this gene. The absence or dysfunction of CFTR results in aberrant ion and liquid homeostasis at epithelial surfaces of the respiratory, intestinal and reproductive tracts as well as other secretory and reabsorptive epithelia.

*ABCC8:* Also known as SUR1 (sulfonylurea receptor 1), ABCC8 protein, as one of the component of ATP-sensitive potassium channels (K(ATP) channels), binds sulfonylurea and regulate potassium channels involved in modulating insulin secretion. Common polymorphisms in these genes have been variably associated with impaired glucose tolerance, type 2 diabetes, predisposed conversion from impaired glucose to type 2 diabetes, and the autosomal recessive form of hyperinsulinism. Some SNPs in ABCC8 gene are also associated with type 2 diabetes and elevated blood pressure levels. ABCC8 has also demonstrated association with familial persistent hyperinsulinemic hypoglycemia of infancy.

*ABCC9:* Also known as SUR2 (sulfonylurea receptor 2), ABCC9 protein is also a component of ATP-sensitive potassium channels in cardiac, skeletal, and vascular and non-vascular smooth muscle. Protein structure suggests a role as the drug-binding channel-modulating subunit of the extrapancreatic ATP-sensitive potassium channels. A recent finding associates sleep duration with ABCC9 gene. Alternative splicing of this gene results in several products, two of which result from differential usage of two terminal exons and one of which results from exon deletion.

*ABCC10:* Also known as MRP7, ABCC10 protein transports amphipathic anions and confers resistance to a variety of agents, such as docetaxel, vincristine and paclitaxel. MRP7 is also able to confer resistance to nucleoside-based agents, such as the anticancer agents cytarabine (Ara-C) and gemcitabine, and the antiviral agents 2',3'-dideoxycytidine and PMEA. In addition, ABCC10 has a broad resistance profile for natural product agents. Unlike MRP1 and MRP2, MRP7-mediated drug transport does not involve glutathione.

*ABCC11:* ABCC11, encoding multidrug resistance protein 8 (MRP8), has been implicated in drug resistance of breast cancer by virtue of its ability to confer resistance to fluoropyrimidines and to efflux methotrexate, and by its expression in breast tumors. ABCC11 expression is negatively regulated by estradiol (E2), but ABCC11 expression is high in high-expressing estrogen receptor-α breast cancers (ER α-positive breast cancers). Expression of ABCC11 was upregulated with the presence of tamoxifen (TAM), and was overexpressed in TAM-resistant cell lines. Therefore, high expressing ABCC11 may contribute to decreased sensitivity to chemotherapy combinations, and may be a potential predictive tool in the choice of anticancer therapies in ER-positive breast cancers resistant to TAM.

*ABCC12:* ABCC12 encodes multidrug resistance protein 9 (MRP9). ABCC11 and ABCC12 are tandemly duplicated on chromosome 16q12. The transcripts of ABCC11 and ABCC12 genes were detected by in various adult human tissues, including liver, lung, and kidney, and also in several fetal tissues. Increased expression of ABCC12 is associated with breast cancer. Further, ABCC11 and ABCC12 were mapped to a region harboring gene(s) for paroxysmal kinesigenic choreoathetosis, and thus the two genes represent positional candidates for this disorder.

*ABCC13:* The ABCC13 gene has a tissue-specific expression, highest in fetal liver, bone marrow, and colon. While ABCC13 was expressed in the bone marrow, its expression in peripheral blood leukocytes of adult humans was much lower and no detectable levels were observed in differentiated hematopoietic cells. The expression of ABCC13 decreased during cell differentiation. These results suggest that the expression of human ABCC13 is related with hematopoiesis. Alternative splicing of ABCC13 results in multiple transcript variants of the gene.

### 4) ABCG subfamily

The human ABCG subfamily includes six "reverse" half transporters that have an NBF at the N terminus and a TM domain at the C terminus, including the closely studied ABCG gene, which is the white locus of Drosophila. In Drosophila, the white protein transports precursors of eye pigments (guanine and tryptophan) in eye cells. Mammalian ABCG1 protein is involved in cholesterol transport regulation. The ABCG subfamily also includes ABCG2 (BCRP), a drug-resistance gene, expressed in intestine, kidney, liver, brain, spleen prostate and other tissues. Other ABCG subfamily members include ABCG3, so far found only in rodents; ABCG4 gene, expressed primarily in liver; ABCG5 and ABCG8, encoding transporters of sterols in the intestine and liver. Exemplary members are detailed below.

*ABCG2:* ABCG2 protein functions as a xenobiotic-transporting ATPase, and has been tested for association to many diseases, such as breast neoplasms, carcinoma, merkel cell, gallbladder neoplasms, leukemia, melanoma and other diseases. ABCG2, also known as BRCP, is a component of MHC class I molecules, which are present on all nucleated cells. The BRCP gene maps to chromosome 4q22 and encodes a half transporter. Cell lines that are resistant to mitoxantrone but do not overexpress ABCB1 or ABCC1 led to the identification of the BRCP gene as a drug transporter. The gene confers resistance to anthracycline anticancer drugs and has been shown to be amplified, or involved in chromosomal translocations, in cell lines that survive exposure to topotecan, mitoxantrone, or doxorubicin. BRCP also transports dyes such as rhodamine and Hoechst 33462. The gene is also expressed in the trophoblast cells of the placenta, and in the intestine. Inhibition of the transporter in the intestine could be useful in making substrates orally available. The evidence supporting the likely involvement of BCRP as one of three major transporter genes involved in drug resistance in mammalian cells indicates that inhibition or inactivation of these ABC transporters may be useful in preventing the development of drug-resistant tumors. In yet another embodiment, the transporter having disrupted expression may be ABCG2.

### (ii) SLC transporter superfamily

The SLC transporter superfamily includes passive transporters, symporters and antiporters, as well as mitochondrial and vesicular transporters. The passive transporters simply move compounds down a concentration gradient while the active transporters utilize an energetically favorable co-substrate (non-**ATP**) to transport a molecule against a concentration gradient. (For reviews, see He L. et al., Analysis and update of the human solute carrier (SLC) gene superfamily, Hum Genomics 3, 195- 206 (2009); and Hediger MA et al., The ABCs of solute carriers: Physiological, pathological and therapeutic implications of human membrane transport proteins, Pfleugers Arch 447, 465-468 (2004)). Non-limiting examples of human SLC transporters relevant to ADME/Tox applications are listed in Table B.

### 1) SLC10 subfamily

The SLC10 subfamily comprises a group of sodium/bile co- transporters. Exemplary members are detailed below.

*SLC10A1:* Also known as NTCP, SLC10A1 encodes a sodium/bile acid cotransporter that participates in the enterohepatic circulation of bile acids, and is found in the basolateral membranes of hepatocytes.

*SLC10A2:* SLC10A2 has common names such as ASBT, IBAT, ISBT, PBAM, NTCP2. SLC1OA2 transporter is the primary mechanism for uptake of
intestinal bile acids by apical cells in the intestinal lumen, the bile duct, and the kidney. Bile acids are the catabolic product of cholesterol metabolism, so this protein is also critical for cholesterol homeostasis. Mutations in this gene cause primary bile acid malabsorption (PBAM); mutations in this gene may also be associated with other diseases of the liver and intestines, such as familial hypertriglyceridemia (FHTG).

### 2) SLC15 subfamily

The SLC subfamily comprises a group of proton-coupled peptide transporters (PEPTs), whose members are best known for their role in nutrient acquisition in the gastrointestinal tract. Peptide transporters are integral plasma membrane proteins that mediate the cellular uptake of dipeptides and tripeptides in addition to a variety of peptidomimetics. The carriers, which occur predominantly in the brush-border membranes of epithelial cells of the small intestine, lung, choroid plexus and kidney, contribute to absorption, distribution and elimination of their substrates. The cellular uptake of peptides and peptidomimetics involves the cotransport of protons down an inwardly directed, electrochemical proton gradient that provides the driving force and causes the electrogenicity of the translocation step. Exemplary members are detailed below.

*SLC15A1:* SLC15A1 encodes PEPT1 protein, which is capable of transporting a broad array of neutral, acidic, and basic di- and tripeptides and peptidomimetic drugs by an H+-dependent, electrochemical active transport mechanism. SLC15A1 is expressed mainly in the intestine, although its expression in kidney and liver has also been detected. Intestinal diseases, such as ulcerative colitis, Crohn's disease, and short-bowel syndrome, are believed to be associated with PEPT1 overexpression in colon. Studies have show that His-57 in PEPT1 is the most critical histidyl residue necessary for the catalytic function of the transporter. Intestinal protein digestion generates a huge variety and quantity of short chain peptides that are absorbed into intestinal epithelial cells by the PEPT1 transporter. Similarly, a variety of drugs with the similar basic structure of di- or tripeptides, such as aminocephalosporins and aminopenicillins, selected angiotensin-converting inhibitors, and amino acid conjugated nucleoside-based antiviral agents are transported by PEPT1. After transport into cells, the small peptides and peptide-like structures primarily undergo intracellular hydrolysis to free amino acids for delivery into portal blood. PEPT1 has been shown to play important roles in nutritional and pharmacological therapies.

*SLC15A2:* SLC15A2 encodes PEPT2 protein, a high-affinity H+/dipeptide transporter expressed in intestine, kidney, brain, lung, liver and mammary gland. PEPT2 is known to have similar but not identical structural requirements for substrate recognition and transport compared to PEPT1. The intestinal carrier PEPT1 has a lower affinity for most substrates than the isoform PEPT2. The physiological role of PEPT2 in kidney is to reabsorb small peptides generated by luminal peptidases. PDZK1 (PDZ domain containing 1) directly interacts with PEPT2, exerting functional regulation of PEPT2 transporting activity. Co-expression of PDZK1 enhancesPEPT2 transporting activity. Studies have shown that His-87 in PEPT2 is the most critical histidyl residue necessary for the catalytic function of the transporter. Moreover, PEPT2 has a significant influence on the in vivo disposition and half-life time of peptide- like drugs within the body, particularly in kidney and brain.

### 3) SLC16 subfamily

This subfamily comprises a group of proton-linked transporters mediating the reversible transport of a wide variety of monocarboxylic acids including lactate, pyruvate, D,L-3-hydroxybutyrate, acetoacetate, alpha-oxoisohexanoate and alpha-oxoisovalerate, ketone bodies, beta-hydroxybutyrate, but not dicarboxylic and tricarboxylic acids. Monocarboxylic acids play a major role in the metabolism of lactic acid. Lactic acid must be rapidly transported into the cells of tissues such as brain, heart, red skeletal muscle and liver, which readily oxidize lactic acid that may become a major respiratory fuel under some conditions. Exemplary members of this family are detailed below.

*SLC16A1:* Also known as MCT1, SLC16A1 is ubiquitously expressed, and is particularly abundant in erythrocytes, cardiac muscle, and basolateral intestinal epithelium. Recent studies demonstrate the involvement of MCT1 in the luminal uptake of short-chain fatty acids (SCFAs) in the human intestine. Significant decline in the expression of MCT1 protein in colonic tissue was observed during transition from normality to malignancy. The decline in MCT1 expression during colon carcinogenesis may reduce the intracellular availability of butyrate required to regulate expression of genes associated with the processes maintaining tissue homeostasis within the colonic mucosa. MCT1 also facilitates the transport of monocarboxylates across cell membranes of the blood-brain barrier and brain parenchymal cells.

*SLC16A7:* SLC16A7 encodes monocarboxylate transporter 2 (MCT2), which is a high affinity pyruvate and lactate transporter. This transporter is inhibited by L-lactate, DL-3-hydroxybutyrate, 3-hydroxybutyrate, and **DL-P** hydroxybutyrate in multiple human tissues including testis, spleen, heart, kidney, pancreas, skeletal muscle, brain, and leukocyte.

*SLC16A8:* SLC16A8 encodes MCT3, a proton-coupled monocarboxylate transporter. MCT3 catalyzes the rapid transport across the plasma membrane of many monocarboxylates such as lactate, pyruvate, branched-chain oxo acids derived from leucine, valine and isoleucine, and the ketone bodies acetoacetate, beta-hydroxybutyrate and acetate.

*SLC16A3:* SLC16A3 encodes monocarboxylate transporter 4 (MOT4) in skeletal muscle, cortex, hippocampus and cerebellum. Obesity leads to changes in muscular MOT4 expression associated with **LDH** isozyme redistribution that contributes to the hyperlactatemia in insulin resistance.

*SLC16A4:* SLC16A4 encodes monocarboxylate transporter 5, which plays important role in cellular glucose metabolism.

*SLC16A5:* SLC16A5 encodes monocarboxylate transporter 6, which transports various drugs but not typical substrates of other MCT isoforms, indicating substrate specificity distinct from those of other MCTs.

### 4) SLC21/SLCO subfamily

This subfamily comprises organic anion transporting polypeptides (OATPs) that are involved in the membrane transport of bile acids, conjugated steroids, thyroid hormone, eicosanoids, peptides, and numerous drugs in many tissues. Exemplary members of this family are detailed below.

*SLCO1A2:* Also known as OATP1A2, SLCO1A2 encodes a protein capable of mediating the cellular uptake of estrogen metabolites. In breast cancer cells OATP1A2 is expressed nearly 10-fold greater than in adjacent healthy breast tissues. The PXR (nuclear receptor, pregnane X receptor) agonist rifampin induces OATP1A2 expression. A PXR response element was identified in the human OATP1A2 promoter, the specificity of PXR-OATP1A2 promoter interaction has been confirmed.

*SLCO1B1:* SLCO1B1, also known as OATP1B1, encodes the organic anion transporting polypeptide in liver and spleen. OATP1B1 has been shown to transport a number of endogenous and exogenous substances, including bile acids, thyroid hormones and methotrexate, and many statins. A number of common variants in SLCO1B1 have been identified that are strongly associated with an increased risk of statin-induced myopathy. The efficacy of statin therapy and the likelihood that patients will experience adverse effects may be predicted by OATP1 B1 variance.

*SLCO3A1:* SLCO3A1 is also known as OATP-D. OATP-D mRNA is abundant mainly in the heart, testis, brain, and some cancer cells. OATP-D plays an important role in translocating prostaglandins in specialized tissues and cells. Whole genome association study identifies polymorph isms in SLCO3A1 associated with attention deficit hyperactivity disorder. OATP-D splice variants exhibit differed tissue distribution, some of which are involved in the regulation of extracellular vasopressin concentration in human brain and influence the neuromodulation of neurotransmission by cerebral neuropeptides such as vasopressin.

*SLCO4A1:* Also known as OATP-E, SLCO4A1 was originally isolated from human brain, but its mRNA is abundant in various peripheral tissues. OATP-E transports 3,3',5-triiodo-L-thyronine, thyronine, and thus plays a role in thyroid hormone metabolism. The Slco4a1 gene is conserved in human, chimpanzee, dog, cow, mouse, chicken, zebrafish, fruit fly, mosquito, and C. elegans..

*SLCO1C1*: Also known as OATP-F, this gene encodes a member
of the organic anion transporter family, mediating the sodium-independent uptake of thyroid hormones in brain tissues. This protein has particularly high affinity for the thyroid hormones thyroxine, tri-iodothyronine and reverse tri-iodothyronine. Polymorphisms in the gene encoding this protein may be associated with fatigue and depression in patients suffering from hyperthyroidism. Alternative splicing results in multiple transcript variants.

*SLCO4C1*: Also known as OATP-H, SLCO41 encodes a human
kidney-specific organic anion transporter. Human OATP-H transports cardiac glycosides (digoxin), thyroid hormone (triiodothyronine), cAMP, and methotrexate in a sodium-independent manner. Overexpression of SLCO4C1 in rat kidney reduced hypertension, cardiomegaly, and inflammation in the setting of renal failure. Some SNPs in SLCO4C1 are significantly associated with increased risk of preeclampsia in women of African descent.

*SLCO1B3:* SLCO18B is also known as OATP1 B3, OATP8, or LST3. OATP1 B3 protein is one of the transporters contributing to the supply of the estrogen precursor estrone-3-sulfate to estrogen-dependent breast cancer cells. SLCO1B3 polymorphism significantly influences plasma mycophenolic acid glucuronide pharmacokinetics in Japanese renal transplant recipients. The insert-variant allele of the SLCO1B3 gene may increase the concentration-to-dose ratio of cardiac glycosides such as digoxin in hemodialysis patients. Some SLCO1B3 variants contribute to mild unconjugated hyperbilirubinemia.

*SLCO2B1:* Also known as OATP-B, SLCO2B1 encodes a protein that may function in regulation of steroid hormone conjugates transport and is expressed in organs with steroidogenic activity, such as placenta, brain and skin. Alternatively spliced transcript variants have been described. OATP-B was identified as a modulator of skeletal muscle statin exposure and toxicity.

### 5) SLC22 subfamily

This subfamily comprises two subgroups: 1) the organic anion transporters (OATs) which are involved in the sodium-dependent transport and excretion of organic anions; and 2) the OCT transporters, which are polyspecific organic cation transporters in the liver, kidney, intestine, and other organs that are critical for elimination of many endogenous small organic cations as well as a wide array of drugs and environmental toxins. Exemplary members of this family are detailed below.

*SLC22A6:* Also known as OAT1, SLC22A6 encodes a protein that is involved in the sodium-dependent transport and excretion of organic anions, some of which are potentially toxic. The encoded protein is an integral membrane protein and may be localized to the basolateral membrane. Four transcript variants encoding four different isoforms have been found for this gene.

*SLC22A7:* Also known as OAT2, SLC22A7 encodes a protein that is an integral membrane protein and appears to be localized to the basolateral membrane of the kidney. Alternatively spliced transcript variants encoding different isoforms have been described. Human OAT2 transporter is a highly efficient, facilitative transporter of cGMP and may be involved in cGMP signaling in many tissues.

*SLC22A8:* Also known as OAT3, like SLC22A7, SLC22A8 encodes a protein that is also an integral membrane protein at the basolateral membrane of the kidney. Human OAT3 transporter is responsible for the basolateral uptake of edaravone sulfate in the kidney, and it also contributes to the renal uptake of rosuvastatin. Rosuvastatin is highly effective in reducing low-density lipoprotein cholesterol. Clinical trials have demonstrated that renal excretion and, in particular, tubular secretion, plays a role in rosuvastatin clearance.

*SLC22A11:* Also known as OAT4, SLC22A11 encodes a transporter protein found mainly in the kidney and in the placenta, where it may act to prevent potentially harmful organic anions from reaching the fetus. Genetic variants of human OAT4 demonstrate altered transport of endogenous substrates. Several naturally occurring SNPs lead to OAT4 variants that may have impaired renal tubular re absorption of important drug substrates. OAT4 mediates high affinity transport of estrone sulfate and dehydroepiandrosterone sulfate. The rapid bone loss that occurs in post-menopausal women is mainly due to a net decrease of estrogen. Studies have shown that OAT4 variants can cause inter-individual variation in anionic drug uptake and, therefore, could be used as markers for certain diseases including osteoporosis.

*SLC22A9:* Also known as OAT7, SLC22A9 encodes an organic anion transporter transporting sulfate-conjugates in exchange for butyrate in hepatocytes.

*SLC22A1:* Also known as OCT1, SLC22A1 encodes a polyspecific organic cation transporter that is expressed in the liver, kidney, intestine, and other organs. The SLC22A1 transporter is critical for elimination of many endogenous small organic cations as well as a wide array of drugs and environmental toxins. Two transcript variants encoding two different isoforms have been found for this gene, but only the longer variant encodes a functional transporter. Human OCT1 transcript levels and single nucleotide polymorphisms are associated with response to imatinib in chronic myeloid leukemia.

*SLC22A2:* Also known as OCT2, this gene is one of three similar cation transporter genes located in a cluster on chromosome 6. The encoded protein contains twelve putative transmembrane domains and is a plasma integral membrane protein. It is found primarily in the kidney, where it may mediate the first step in cation reabsorption. Some SNPs have been identified that may affect creatinine production and secretion.

*SLC22A3:* SLC22A3 is also known as OCT3. The functional properties of single nucleotide polymorph isms (SNPs) in OCT3 gene (SLC22A3) have been analyzed. Some SNPs exhibited reduced uptake of substrates, and differ in certain disease processes such as hypertension, allergic diseases, and neuropsychiatric diseases by the clearance of endogenous organic cations such as biogenic amines.

*SLC22A4:* SLC22A4 is also known as OCTN1. The encoded protein is an organic cation transporter and plasma integral membrane protein containing eleven putative transmembrane domains as well as a nucleotide-binding site motif. Transport by this protein is at least partially ATP-dependent. One SNP of SLC22A4 is associated with Crohn's disease. OCTN1 plays a pivotal role for maintenance of systemic and intestinal exposure of ergothioneine and may provide a possible diagnostic tool for distinguishing inflammatory bowel diseases.

*SLC22A5:* SLC22A5 is also known as OCTN2. The encoded protein is a plasma integral membrane protein which functions both as an organic cation transporter and as a sodium-dependent high affinity carnitine transporter. The encoded protein is involved in the active cellular uptake of carnitine. Mutations in this gene are associated with systemic primary carnitine deficiency (CDSP), an autosomal recessive disorder manifested early in life by hypoketotic hypoglycemia and acute metabolic decompensation, and later in life by skeletal myopathy or cardiomyopathy.

### 6) SLC47 subfamily

The SLC47 subfamily comprises a group of transporters involved in the excretion of toxic electrolytes, both endogenous and exogenous, through urine and bile. Exemplary members are detailed below.

*SLC47A1:* Also known as MATE1 (multidrug and toxin extrusion 1), this gene is located within the Smith-Magenis syndrome region on chromosome 17.

*SLC47A2:* Like SLC47A1, SLC47A2 is responsible for drug resistance. Alternatively spliced transcript variants encoding different isoforms have been identified for this gene.

### 7) Miscellaneous transporters

*OSTα-OST*β: The organic solute transporter (OSTalpha-OSTbeta) is a heteromeric transporter that is expressed on the basolateral membrane of epithelium in intestine, kidney, liver, testis and adrenal gland and facilitates efflux of bile acids and other steroid solutes. Both subunits are required for plasma membrane localization of the functional transporter.

### (b) Drug Metabolizing Enzymes

An ADME/Tox protein may be a drug metabolizing enzymes (DME). Drug metabolizing enzymes (DMEs) facilitate the conversion of xenobiotics to hydrophilic molecules more suitable for excretion into urine or bile. Drug metabolism is mediated primarily by oxidation, reduction, hydrolysis, or conjugation enzymes, and directly affects drug effects and toxicity. The drug metabolizing enzymes are divided into two groups: phase I enzymes and phase II enzymes.

Phase I enzymes are oxidative drug metabolizing enzymes that include cytochrome p450s (CYPs) and flavin monooxygenases (FMOs), which catalyze the introduction of an oxygen atom into substrate molecules, generally resulting in hydroxylation or demethylation. Non-limiting examples of Phase I enzymes relevant for ADME/Tox applications are provided in Table C.

| TABLE C. Human Phase I Enzymes | | | |
|---|---|---|---|
| Gene symbol | Gene common name | UniProtKB/ Swiss-Prot Access No. | Protein Annotation |
| CYP1A1 | CYP1A1 | P04798 | Cytochrome p450 1A1 |
| CYP1A2 | CYP1A2 | P05177 | Cytochrome p450 1A2 |
| CYP2A6 | CYP2A6 | P11509 | Cytochrome p450 2A6 |
| CYP2B6 | CYP2B6 | P20813 | Cytochrome p450 2B6 |
| CYP2C8 | CYP2C8 | P10632 | Cytochrome p450 2C8 |
| CYP2C9 | CYP2C9 | P11712 | Cytochrome p450 2C9 |
| CYP2C19 | CYP2C19 | P33261 | Cytochrome p450 2C19 |
| CYP2D6 | CYP2D6 | Q5Y7H2 | Cytochrome p450 2D6 |
| CYP2E1 | CYP2E1 | P05181 | Cytochrome p450 2E1 |
| CYP3A4 | CYP3A4 | P08684 | Cytochrome p450 3A4 |
| CYP3A5 | CYP3A5 | P20815 | Cytochrome p450 3A5 |
| CYP3A7 | CYP3A7 | P24462 | Cytochrome p450 3A7 |
| POR | POR | P16435 | Cytochrome p450 oxidoreductase |
| FMO1 | FMO1 | Q01740 | Flavin containing monooxygenase 1 |
| FMO2 | FMO2 | Q99518 | Flavin containing monooxygenase 2 |
| FMO3 | FMO3 | P31513 | Flavin containing monooxygenase 3 |
| FMO4 | FMO4 | P31512 | Flavin containing monooxygenase 4 |
| FMO5 | FMO5 | P49326 | Flavin containing monooxygenase 5 |
| ADH | ADH | P07327 | Alcohol dehydrogenase |
| AOX | AOX | Q06278 | Aldehyde oxidase |
| ALDH3A1 | ALDH | P30838 | Aldehyde dehydrogenase |
| ALDH3A2 | ALDH | P51648 | Fatty aldehyde dehydrogenase |
| AKR | AKR | P14550 | Aldo-keto reductase |
| CES1 | CES | P23141 | Carboxylesterase 1 |
| CES2 | CES | O00748 | Carboxylesterase 2 |
| EPHX1 | EPHX | P07099 | Epoxide hydrolase (microsomal) |
| EPHX2 | EPHX | P34913 | Epoxide hydrolase (cytoplasmic) |
| MAOA | MAO-A | P21397 | Monoamine oxidase A |
| MAOB | MAO-B | P27338 | Monoamine oxidase B |
| MPO | MPO | P05164 | Myeloperoxidase |
| PTGS2 | COX-2 | P35354 | Prostaglandin endoperoxide synthase 2 |
| LPO | LPO | P22079 | Lactoperoxidase |

Phase II enzymes are conjugative enzyme families that include UDP-glucuronosyltransferases (UGTs), glutathione transferases (GSTs), sulfotransferases (SULTs), N-acetyltransferases (NATs), and methyltransferases, which catalyze the coupling of endogenous small molecules to xenobiotics that usually results in the formation of soluble compounds that are more readily excreted. (For a review, see: Jancova et al. Phase II metabolizing enzymes, Biomed Pap Med Fac Univ Palacky Olomouc Czech Repub 154, 103-116 (2010)). Non-limiting examples of Phase II enzymes relevant for ADME/Tox applications are provided in Table D.

| **TABLE D. Human Phase II Enzymes** | | | |
|---|---|---|---|
| **Gene symbol** | **Gene common name** | **UniProtKB/ Swiss-Prot Access No.** | **Protein Annotation** |
| UGT1A1 | UGT1A1 | P22309 | UDP-glucuronosyl transferase 1A1 |
| UGT1A3 | UGT1A3 | P35503 | UDP-glucuronosyl transferase 1A3 |
| UGT1A4 | UGT1A4 | P22310 | UDP-glucuronosyl transferase 1A4 |
| UGT1A6 | UGT1A6 | P19224 | UDP-glucuronosyl transferase 1A6 |
| UGT1A9 | UGT1A9 | O60656 | UDP-glucuronosyl transferase 1A9 |
| UGT2B7 | UGT2B7 | P16662 | UDP-glucuronosyl transferase 2B7 |
| UGT2B15 | UGT2B15 | P54855 | UDP-glucuronosyl transferase 2B15 |
| SULT1A1 | SULT1A1 | P50225 | Sulfotransferase 1A1 |
| SULT1A2 | SULT1A2 | P50226 | Sulfotransferase 1A2 |
| SULT1A3 | SULT1A3 | P50224 | Sulfotransferase 1A3 |
| SULT1B1 | SULT1B1 | O43704 | Sulfotransferase 1B1 |
| SULT1C2 | SULT1C2 | O00338 | Sulfotransferase 1C2 |
| SULT1E1 | SULT1E1 | P49888 | Sulfotransferase 1E1 |
| SULT2A1 | SULT2A1 | Q06520 | Sulfotransferase 2A1 |
| NAT1 | NAT-1 | P18440 | N-acetyltransferase 1 |
| NAT2 | NAT-2 | P11245 | N-acetyltransferase 2 |
| TPMT | TPMT | P51580 | Thiopurine S-methyl transferase |
| COMT | COMT | P21964 | Catechol O-methyl transferase |
| GSTA1 | GSTA1 | P08263 | Glutathione S-transferase A1 |
| GSTA2 | GSTA2 | P09210 | Glutathione S-transferase A2 |
| GSTA3 | GSTA3 | Q16772 | Glutathione S-transferase A3 |
| GSTA4 | GSTA4 | O15217 | Glutathione S-transferase A4 |
| GSTK1 | GSTK1 | Q972Q3 | Glutathione S-transferase K1 |
| GSTM1 | GSTM1 | P09488 | Glutathione S-transferase M1 |
| GSTM2 | GSTM2 | P28161 | Glutathione S-transferase M2 |
| GSTM3 | GSTM3 | P21266 | Glutathione S-transferase M3 |
| GSTM4 | GSTM4 | Q03013 | Glutathione S-transferase M4 |
| GSTM5 | GSTM5 | P46439 | Glutathione S-transferase M5 |
| GSTP1 | GSTP1 | P09211 | Glutathione S-transferase P1 |
| GSTT1 | GSTT1 | P30711 | Glutathione S-transferase T1 |
| GSTT2 | GSTT2 | P0CG30 | Glutathione S-transferase T2 |

Drug metabolizing enzymes thus play complementary roles with transporters in drug disposition by mediating xenobiotics influx into the cell or extrusion to the extracellular space via catalyzing metabolic biotransformations. As detailed below in section (II), genetically modified cells comprising DMEs with disrupted expression, whether with no expression, overexpression, or under-expression, and transporters with disrupted expression provide a powerful tool facilitating inhibitor screening, determining of the kinetic parameter of an agent, and metabolite toxicity evaluation.

### (i) Phase I enzymes

Phase I enzymes include cytochrome p450 (CYP) enzymes, flavin-containing monooxygenase (FMO) enzymes, alcohol/aldehyde metabolism enzymes, carboxylesterases (CES), epoxide hydrolases (EPHX), monoamine oxidases (MAO), and peroxidases, which are detailed below.

### 1) Cytochrome P450 enzymes

Cytochrome p450 (CYP) enzymes are a diverse group of catalysts that contains 57 members in humans. CYPs are usually membrane-bound and are localized to the inner mitochondrial or endoplasmic reticular membrane. CYPs have oxygenase activity and commonly catalyze redox reactions, involving the oxidation of the substrate and reduction of water. Enzymes of this group contain a heme ion within the active site, which is essential for catalytic activity. CYPs have been found in all organisms tested and are ubiquitously expressed. They are found at high levels in the liver, where they have an important role in metabolism of drugs and endogenous toxic compounds (for example bilirubin). Most CYPs can metabolize numerous substrates and this accounts for their major role in drug interactions. CYPs also have functions in steroid hormone synthesis, cholesterol synthesis and vitamin D metabolism. Exemplary CYPs are detailed below.

*CYP1A2:* Caffeine is metabolized primarily by CYP1A2 in the liver through an initial N3-demethylation. CYP1A2 also acts in the metabolism of aflatoxin B1 and acetaminophen. CYP1A2 participates in the bioactivation of carcinogenic aromatic and heterocyclic amines. CYP1A2 catalyzes the N-hydroxylation of heterocyclic amines and the O-demethylation of phenacetin. Large interindividual differences are seen in the levels of this enzyme due to the distribution of several different polymorphic gene variants mainly located in the open reading frame (ORF).

*CYP2A6:* CYP2A6 constitutes the major nicotine C-oxidase. It exhibits a high coumarin 7-hydroxylase activity. It acts in the hydroxylation of the anti cancer drugs cyclophosphamide and ifosphamide. It is competent in the metabolic activation of aflatoxin B1 and it possesses low phenacetin O-demethylation activity.

*CYP2B6:* This protein localizes to the endoplasmic reticulum and its expression is induced by phenobarbital. CYP2B6 is involved in an NADPH dependent electron transport pathway. The enzyme is known to metabolize some xenobiotics, such as the anti-cancer drugs cyclophosphamide and ifosphamide. Transcript variants for this gene have been described.

*CYP2C8:* The enzyme is known to metabolize many xenobiotics, including the anticonvulsive drug mephenytoin, benzo(a)pyrene, 7- ethyoxycoumarin. It is the principal enzyme responsible for the metabolism the anti- cancer drug paclitaxel (taxol). This gene is located within a cluster of cytochrome P450 genes on chromosome 10q24. Several transcript variants encoding a few different isoforms have been found for this gene.

*CYP2C9:* This enzyme is known to metabolize many xenobiotics, including phenytoin, tolbutamide, ibuprofen and S-warfarin. CYP2C9 contributes to the wide pharmacokinetics variability of the metabolism of drugs such as S-warfarin, diclofenac, phenytoin, tolbutamide and losartan, suggesting this gene is polymorphic. The gene is located within a cluster of cytochrome P450 genes on chromosome 1Oq24.

*CYP2C19:* CYP2C19 metabolize many xenobiotics, including the anticonvulsive drug mephenytoin, omeprazole, diazepam and some barbiturates. Polymorphism within this gene is associated with the variable ability to metabolize mephenytoin, known as the poor metabolizer and extensive metabolizer phenotypes. The gene is located within a cluster of cytochrome P450 genes on chromosome 10q24.

*CYP2D6:* This protein localizes to the endoplasmic reticulum and is known to metabolize as many as 20% of commonly prescribed drugs. Its substrates include debrisoquine, an adrenergic-blocking drug; sparteine and propafenone, both anti-arrythmic drugs; and amitryptiline, an anti-depressant. The gene is highly polymorphic in the population; certain alleles result in the poor metabolizer phenotype, characterized by a decreased ability to metabolize the enzyme's substrates. The gene is located near two cytochrome P450 pseudogenes on chromosome 22q13.1. Alternatively spliced transcript variants encoding different isoforms have been found for this gene.

*CYP2E1:* CYP2E1 is the rate-limiting cytochrome P450 enzyme that initiates the cascade of events leading to acetaminophen hepatotoxicity. In the absence of CYP2E1, toxicity will only be apparent at high concentrations. CYP2E1 is induced by alcohol and the primary P450 that carries out ethanol oxidation that can lead to the production of activated oxygen species and oxidative stress. Activation of this pathway inhibits apoptotic cell death stimulated by reactive oxygen generating chemicals but accelerates necrotic cell death produced by polyunsaturated fatty acids.

*CYP3A*/*3A5:* CYP3A4 is the most abundantly expressed member of the cytochrome P450 family, and has been implicated in the metabolism of more than 50% of prescribed pharmaceuticals. GYP3A4 is highly expressed in key sites of drug disposition, such as liver and small intestine. However, the interindividual variability of CYP3A4 expression and/or function has been estimated to be between 5- and 20-fold. CYP3A4 expression is regulated by a number of nuclear receptors. CYP3A5 is the predominant form expressed in extrahepatic tissues.

*CYP3A7:* CYP3A7 is a member of the CYP3A family. CYP3A7 is the major fetal form and is rarely expressed in adults.

*Cytochrome p450 oxidoreductase (POR):* POR is an oxidoreductase that provides reducing equivalents for all CYP enzymes. It is essential to the function of GYPs and therefore is an attractive target for global inhibition of CYP activity.

### 2) Flavin-containing monooxygenases

Flavin-containing monooxygenases (FMOs) catalyze the oxidation of heteroatoms, particularly nucleophilic atoms such as the nitrogen of amines, or sulfur and phosphorus heteroatoms of a variety of xenobiotics. Similar to cytochrome p450, FMOs require NADPH and oxygen but utilizes a different catalytic mechanism involving the flavin moiety. They are also not under the same regulatory mechanisms as CYPs. FMO3 is the major human liver isoform, while FMO1 is the predominant form in the kidney.

*FMO1:* FMO1 is the major FMO in human kidney; however it is only expressed at very low levels in the liver. FMO1 has a substrate specificity that is distinct from that of FMO3, whereas FMO1 is only highly efficient at N-oxygenating tertiary amines. Both FMO1 and FMO3 S-oxygenate a number of nucleophilic sulfur containing substrates.

*FMO3:* FMO3 is the major FMO in human liver microsomes. This isoform is predominantly if not solely responsible for converting (S)-nicotine to (S) nicotine N-1'-oxide and is also the principal enzyme involved in the S-oxygenation of cimetidine, an older H2-antagonist commonly used to treat gastric ulcers. N-oxidation of a diet-derived amino-trimethylamine (TMA) is catalyzed by FMO3 and humans with an inherited polymorphism in this gene have reduced activity toward this substrate resulting in a rare fish odor syndrome termed trimethylaminuria.

### 3) Alcohol/Aldehyde metabolism enzymes

*Alcohol dehydrogenase (ADH):* ADH catalyzes the oxidation of primary and secondary alcohols to aldehydes and ketones, respectively.

*Aldehyde oxidase (AOX):* AOX is an enzyme that generates carboxylic acids from aldehydes. In is present in human liver and it is involved in the metabolism of a number of drugs, including famciclovir. In addition, the enzyme is also subject to inhibition by many commonly prescribed drugs and thus is a potential source for adverse drug-drug interactions.

*Aldehyde dehydrogenase (ALDH):* ALDH is a polymorphic family of enzymes that also catalyzes the oxidation of aldehydes to carboxylic acids. There are three different classes of ALDH which all include constitutive and inducible forms. The most common isoforms in human liver are ALDH1 and ALDH2. Fatty aldehyde dehydrogenase is an ALDH enzyme associated with the Sjogren-Larsson syndrome.

*Aldo-Keto reductase (AKR):* Also known as aldehyde reductase, this enzyme is involved in the reduction of biogenic and xenobiotic aldehydes and is present in virtually every tissue.

### 4) Carboxylesterases

Mammalian carboxylesterases (CES) represent a multigene family whose members are widely distributed in diverse tissues. These enzymes are major determinants of the pharmacokinetic behavior of most therapeutic drugs containing ester or amide bonds. In addition, many pro-drugs are specifically designed to become activated when cleaved by carboxylesterase activity.

### 5) Epoxide hydrolases

Epoxide hydrolases (EPHX) convert arene and alkene oxides (epoxides) to dihydrodiols via the addition of water. Epoxides are important in toxicology as they have known mutagenic and carcinogenic properties. Although the hydrolysis of epoxides is generally thought to be a detoxification reaction, in some cases (e.g. benzo[a]pyrene), the metabolites of epoxides are even more mutagenic and carcinogenic. Humans express four isozymes of epoxide hydrolase, located in both the microsomal and cytosolic cellular compartments.

### 6) Monoamine oxidases

Monoamine oxidases (MAO) are flavoproteins located in the mitochondrial fraction of liver, kidney and brain. They catalyze the deamination of primary, secondary, and tertiary aliphatic amines. There are two main types of MAO in humans - MAO-A and MAO-B. MAOs play an important role in the inactivation of neurotransmitters, and numerous drugs have been developed to inhibit their activity. MAO-A inhibitors are used as antidepressants and antianxiety agents, while MAO-B inhibitors are used alone or in combination to treat Alzheimer's and Parkinsons disease.

### 7) Peroxidases

Peroxidases catalyze the reduction of hydrogen peroxide or organic hydroperoxides. There are a large number of mammalian peroxidases, including myeloperoxidase, lactoperoxidase and prostaglandin endoperoxide synthase (PHS or COX-2). The reduction of peroxides is coupled with the oxidation of a donor molecule, which can either be an endogenous molecule or a variety of xenobiotic compounds. The oxidation of xenobiotics is often termed cooxidation and has been widely documented. For example, the cooxidation of aromatic amines by PHS to potentially toxic nitrogen- and oxygen-centered free radicals has been demonstrated. The ultimate importance of peroxidase-catalyzed reactions in the overall biotransformation and toxicity of drugs, however, remains largely unknown.

### (ii) Phase II enzymes

As detailed above, phase II enzymes include glutathione transferases, UDP-glucuronosyltransferases, sulfotransferases, N-acetyltransferases, and methyltransferases.

### 1) Glutathione 5-transferases

Cytoplasmic and membrane-bound forms of glutathione S-transferase (GST) are encoded by two distinct supergene families. GST enzymes function in the detoxification of electrophilic compounds, including carcinogens, therapeutic drugs, environmental toxins and products of oxidative stress, by conjugation with glutathione. At present, eight distinct classes of the soluble cytoplasmic mammalian glutathione S-transferases have been identified: alpha, kappa, mu, omega, pi, sigma, theta and zeta. Non-limiting examples of GSTs relevant for ADME/Tox applications are detailed below.

*GSTA1:* This gene encodes a glutathione S-transferase belonging to the alpha class. The alpha class genes, located in a cluster mapped to chromosome 6, are the most abundantly expressed glutathione S-transferases in liver. In addition to metabolizing bilirubin and certain anti-cancer drugs in the liver, the alpha class of these enzymes exhibit glutathione peroxidase activity thereby protecting the cells from reactive oxygen species and the products of peroxidation.

*GSTA2:* The protective adaptive response to electrophiles and reactive oxygen species is mediated by the induction of phase II detoxifying genes including glutathione S-transferases (GSTs). GSTA2 gene expression is induced by oxidative stress via Nrf2 (NF-E2-related factor-2). Nitric oxide synthase in hepatocytes is required for GSTA2 induction.

*GSTA4:* Overexpression of glutathione-S-transferees (GSTs) in endothelium protects against oxidative damage from aldehydes such as 4-HNE. GSTA4 may play an important defensive role against atherogenesis through detoxification of 4-HNE and upregulation of iNOS (inducible nitric oxide synthase). 4-HNE induces apoptosis and triggers the translocation of NF-kappaB, and thus upregulates the NF-kappaB mediated gene expression, such as iNOS.

*GSTM1:* GSTM1 is a member of the mu subfamily of the glutathione S-transferase multigene family. Both GSTT1 (theta subfamily member) and GSTM1 are candidate cancer susceptibility genes because of their ability to regulate the conjugation of carcinogenic compounds to excretable hydrophilic metabolites. Deletion variants that are associated with a lack of enzyme function exist at both these loci. Individuals who are carriers of homozygous deletions in the GSTM1 or GSTT1 genes may have an impaired ability to metabolically eliminate carcinogenic compounds and may therefore be at increased cancer risk. The relationship of GSTM1 and GSTT1 affects cancer susceptibility. The frequencies of homozygous GSTM1 and GSTT1 deletion carriers is very high (i.e., 20-50%) in most populations. Further, GSTM1 and, possibly, GSTT1 may be involved in the etiology of cancer at more than one site.

### 2) UDP-Glucuronosyltransferases

UDP-glucuronosyltransferases (UGT) are membrane-bound enzymes that catalyze the addition of glucuronic acid to hydroxyl, amino and thiol groups. Substrates of these enzymes include endogenous molecules such as steroids, bilirubin, hormones, and many drugs, typically converting them into water-soluble, excretable metabolites (β -D glucuronides). Four UGTfamilies have been identified in humans (UGT1, UGT2, UGT3 and UGT8) with 22 individual enzymes described to date. The most important drug conjugating UGTs belong the UGT1 and UGT2 families. Many of these forms have broad tissue distribution with liver as a major location. In particular, the UGT1A1, 1A3, 1A4, 1A6, 1A9, 287 and 2B15 enzymes are considered to be the most important human liver UGTs. In contrast, UGT1A7, 1A8 and 1A10 are expressed mainly in the gastrointestinal tract. Non-limiting examples of UGTs relevant for ADME/Tox applications are detailed below.

*UGT1A1:* UGT1A1 is the only isoform responsible for bilirubin glucuronidation. It also exhibits moderate activity in the conjugation of simple phenols, anthraquinones/flavonones and C18 steroids and low activity in the conjugation of complex phenols and coumarins. The UGT1A1 gene consists of at least nine promoters and first exons (first exons 3, 11 and 12 are pseudogenes) that can be spliced with four common exons to result in nine different UGT1A1 enzymes. More than 50 genetic variations in the promoter and coding regions of the gene are currently known to decrease the enzyme activity, leading to constitutional unconjugated jaundice (Crigler-Najjar or Gilbert's syndromes). UGT1A1 is well-known to play a role in the bone marrow and gastrointestinal side effects of the anticancer drug, irinotecan (CPT-11). Irinotecan is mainly eliminated unchanged by the liver and to a minor extent by the kidneys. In liver, the drug can be converted into an inactive metabolite by CYP3A4 and into an active metabolite, SN-38, by carboxylesterase enzymes. SN-38 is further metabolized by UGT1A1 into SN-38 glucuronide (SN-38G). High concentrations of SN-38 in blood are associated with toxicity.

*UGT1A3:* One of the major UGTs involved in the conjugation of carboxylic acids.

*UGT2B7:* UGT2B7 is a member of the UGT2B family that conjugates steroid hormones as well as bile acids and xenobiotics. UGT2Bs are expressed in numerous human tissues, such as skin, breast, prostate, adipose, and intestine and are hypothesized to modulate steroid metabolism and excretion. UGT2B7 is the major enzyme responsible for the glucuronidation of opioids. Polymorph isms have been identified that may modify substrate specificities or enzyme activities of UGT2B family isozymes.

### 3) Sulfotransferases

Sulfotransferase (SULT) enzymes catalyze the sulfate conjugation of many hormones, neurotransmitters, drugs, and xenobiotic compounds. These reactions can occur with an O-, N- or S-acceptor group of an appropriate molecule, although O-sulfation represents the dominant sulfonation reaction. The sulfur donor is typically 3'-phosphoadenosine 5'-phosphosulfate (PAPS). To date, four human SULT families have been identified with at least 13 distinct members. These cytosolic enzymes differ in their tissue distribution and substrate specificities. The gene structure (number and length of exons) is similar among family members. Non-limiting examples of SULTs relevant for ADME/Tox applications are detailed below.

*SULT1A1:* This enzyme exhibits the highest expression of the SULT1 enzymes in the liver. SULT1A1 has a broad substrate range and is responsible for the sulfation of numerous phenolic compounds. It has also been termed phenol sulfotransferase and thermostable phenol sulfotransferase. A common genetic polymorphism (25-36% in Caucasians) is known for SULT1A1 in which Arg213 is replaced with His. Patients homozygous for His at this site have significantly reduced platelet sulfotransferase activity.

*SULT1A2:* SULT1A2 is responsible for the sulfonation of several aromatic hydoxylamines. Charged species formed in this reaction (the sulfoconjugates of hydroxylamines) are chemically reactive and mutagenic.

*SULT1A3:* Previously known as thermolabile phenol SULT and monoamine sulfotransferase. It displays high affinity for monocyclic phenols. SULT1A3 is expressed in most adult tissues with the exception of the liver. SULT1A3 also plays a role in sulfonation of catecholamines and thus helps regulate the levels of neurotransmitters. Dopamine is often used as a selective probe for the activity of SULT1A3.

*SULT181:* Substrate specificity of SULT1 B1 is restricted to thyroid hormones and small phenolic compounds such as 1-naphthol and 4-nitrophenol.

*SULT1E1:* Also called estrogen sulfotransferase. This enzyme has a greater affinity for estrogen than any other SULTs. SULT1 E1 may be important in both the metabolism of estrogens and in the regulation of their activities. This enzyme also shows activity towards iodothyronines, pregnenolone, 1-naphthol, naringenin, genistein and 4-hydroxytamoxifen.

### 4) N-Acetyltransferases

There are two arylamine N-acetyltransferase (NAT) genes in the human genome, NAT1 and NAT2. The enzymes encoded by these two genes catalyze the transfer of an acetyl group from acetyl-CoA to various arylamine and hydrazine substrates. This enzyme helps metabolize drugs and other xenobiotics, and functions in folate catabolism. Multiple transcript variants encoding different isoforms have been found for this gene.

*NAT1:* Arylamine N-acetyltransferase-1 (NAT1) has been associated with disorders involving folate metabolism, such as spina bifida, as well as numerous human cancers. In addition to the transcriptional and post-transcriptional regulation of NAT1 activity, histone deacetylase inhibitor trichostatin A (TSA) increases NAT1 activity in human cancer cells through epigenetic regulation. TSA increased the acetylation of histones associated with the NAT1 proximate NATb promoter. This allowed recruitment of Sp1, a transcription factor to the promoter along with acetylated histones. NAT1 transcription is partially repressed by the local chromatin condensation in the vicinity of NATb and that histone deacetylase inhibition leads to up-regulation of NAT1 expression via a direct change in chromatin conformation.

*NAT2:* Arylamine N-acetyltransferase-2 (NAT2) is also an important enzyme in the biotransformation of carcinogens and exhibit genetic polymorphism. For cancers in which N-acetylation is a detoxification step such as aromatic amine-related urinary bladder cancer, the NAT2 slow acetylator phenotype is at higher risk. Multiple studies have shown that the urinary bladder cancer risk is particularly high in the slowest NAT2 acetylator phenotype or genotype (NAT2(*)5). In contrast, for cancers in which N-acetylation is negligible and O-acetylation is an activation step such as for heterocyclic amine-related colon cancer, the NAT2 rapid acetylator phenotype is at higher risk. In contrast, the associations between NAT1 genotype and various cancers are less consistent.

### 5) Methyltransferases

*Catechol O-methyltransferase (COMT):* COMT catalyzes the addition of a methyl group to one of the hydroxyl groups of catecholamines, using S adenosylmethionine as the methyl donor. The enzyme methylates both endogenous and xenobiotic substrates. Examples of endogenous substrates include the neurotransmitters dopamine, epinephrine and norepinephrine.

*Thiopurine S-methyl transferase (TPMT):* TPMT catalyzes the methylation of thiopurine compounds, including thiopurine anti-cancer drugs such as 6-mercaptopurine, azathiopurine and 6-thioguanine. Genetic polymorphisms that affect this enzymatic activity are correlated with variations in sensitivity and toxicity to these drugs within individuals. For example, decreased activity of TMPT causes an accumulation of thiopurine nucleotides and correlates with enhanced bone marrow toxicity.

### (c) Xenobiotic Sensors

In other embodiments, the ADME/Tox protein with disrupted expression may be a xenobiotic sensor. Nuclear receptors are the largest known family of transcription factors that function as modulators of tissue specific gene expression. There are 49 known members of the nuclear receptor superfamily. When a ligand, such as a hormone, bile acid, lipid, eicosanoid, drug, or other xenobiotic enters the cell and binds to a cytosolic nuclear receptor, the receptor is activated. Once activated, the ligand-nuclear receptor complex then translocates to the nucleus, where it recruits coactivators and binds to another nuclear receptor forming a homodimer or heterodimer. The resulting dimeric complex binds to regulatory regions of target genes where it can modulate transcriptional activity. Therefore, nuclear receptors also serve as xenobiotic sensors. Moreover, the ligand activated transcription factors within the nuclear receptor superfamily are critical to the coordinate expression of DMEs and drug transporters in organs such as intestine, liver, and kidney.

The nuclear receptors (e.g. AhR, PXR, and CAR) and cell specific factors (e.g., HNF4α) are required for enzyme induction, inducing both the expression and activities of CYPs as well as phase II enzymes and transporters. For example, the expression of CYP3A4, the most abundantly expressed member of DME cytochrome p450 family in liver and small intestine, is induced by a number of nuclear receptors, including the pregnane X receptor (PXR), constitutive androstane receptor (CAR), the glucocorticoid receptor (GR), hepatocyte nuclear factor 4α (HNF4α), farnesoid X receptor (FXR), and the vitamin D receptor (VDR). Other nuclear receptors include but not limited to liver X receptor (LXR), and peroxisome proliferator-activated receptor (PPAR) (see Table E).

| **TABLE E.** Human Xenobiotic Sensors | | | |
|---|---|---|---|
| Gene symbol | Gene commo n name | UniProtKB/ Swiss-Prot Access No. | Protein Annotation |
| AHR | AhR | P35869 | Aryl hydrocarbon receptor |
| NR112 | PXR | O75469 | Pre nenolone X receptor |
| NR113 | CAR | Q4U0F0 | Constitutive androstane receptor |
| NR1H3 | LXR | Q13133 | Liver X receptor |
| NR1H4 | FXR | Q96RI1 | Farnesyl X receptor |
| PPARA | PPAR | Q07869 | Peroxisome proliferator-activated |

*AhR:* Encoded by the AHR gene, AhR is a ligand-activated transcription factor that controls several dozen genes, including up-regulation of all three CYP1 genes, CYP1A1, CYP1A2 and CYP1B1. Ligands for the AhR include dioxin, PAHs (polycyclic aromatic hydrocarbons), polyhalogenated aromatic hydrocarbons, indoles and tryptophan-derived endogenous ligands, and benzoflavones found especially in cruciferous plants. The AHR participates in cell cycle control and apoptosis that is cell type- or tissue-specific. At least nine mutations in and near the human AHR gene have been found.

*PXR:* Encoded by NR1I2, PXR serves as a generalized sensor of hydrophobic toxins (in contrast to other nuclear receptors that interact selectively with their physiological ligands). PXR binds as a heterodimer with the retinoic acid receptor (NR2B) to DNA response elements in the regulatory regions of cytochrome p450 3A monooxygenase genes and a number of other genes involved in the metabolism and elimination of xenobiotics from the body. PXR is activated by the spectrum of chemicals that are known to induce CYP3A gene expression. Inflammatory bowel disease (IBD) is a complex trait derived from the interaction of genetic and environmental factors, most probably part of the luminal bacterial flora, leading to uncontrolled immune activation and chronic inflammation. Expression of PXR is significantly reduced in the colon of patients with ulcerative colitis (UC), but unaffected in patients with Crohn's disease (CD). Further, the elucidation of the three-dimensional structure of the PXR ligand binding domain (LBO) suggests the structural basis for the promiscuous ligand binding properties of this unusual nuclear receptor.

*CAR:* CAR and PXR are members of the same nuclear receptor subfamily (NR1), sharing about 40% amino acid identity in their ligand binding domains (LBDs). CAR is encoded by NR1I3 and is most abundantly expressed in liver and has strong constitutive activity in cell-based reporter assays in the absence of any added ligand. This constitutive activity can be inhibited by superphysiological concentrations of the testosterone metabolites androstanol and androstenol. These androstanes inhibit the interaction of CAR with the steroid receptor coactivator 1 (SRC-1), suggesting that "deactivation" is mediated by direct binding to the orphan receptor. CAR is not present in the nucleus of primary hepatocytes but is instead sequestered in the cytoplasm.

*LXR:* There are two LXR isoforms in mammals, termed LXRa (NR1 H3) and LXRb (NR1 H2). LXRa is abundantly expressed mainly in the liver, intestine, kidney, spleen, and adipose tissue, whereas LXRb is ubiquitously expressed at a lower level. The two isoforms share almost 80% identity of their amino-acid sequences. After ligand binding, LXRs regulate gene transcription. The LXRs operate as cholesterol sensors which protect from cholesterol overload by inhibiting intestinal cholesterol absorption, stimulating cholesterol efflux from cells to high-density lipoproteins through the ATP-binding cassette transporters ABCA1 and ABCG1, activating the conversion of cholesterol to bile acids in the liver, and activating biliary cholesterol and bile acid excretion. Activation of LXRs inhibits inflammation and autoimmune reactions. Moreover, pharmacological studies and genetic manipulations indicate that these receptors inhibit atherogenesis. LX receptors are also involved in the regulation of renin secretion, inhibit the formation of amyloid beta in the central nervous system, regulate gonadal function and steroidogenesis both in the gonads and adrenals, influence the proliferation and differentiation of keratinocytes, and inhibit the proliferation of tumor cells. Changes in the expression of these receptors and in the level of their agonists are observed in many diseases. Taking into account the multiple roles of LX receptors, their agonists may be applied in the future in the treatment of
many disorders, including diabetes, inflammatory diseases, atherosclerosis, Alzheimer's disease, and hypogonadism.

### (d) Stress Response Pathway Proteins

In still other embodiments, the ADME/Tox protein may be involved in a cellular stress response pathway. Examples of cellular stress response pathways include heat shock, hypoxia, endoplasmic reticulum (ER) stress, DNA damage, oxidative, inflammation, metal stress, and osmotic stress. Investigating cellular stress responses facilitates the testing of drug and environmental chemical behavior and toxicity in the process of absorption, metabolism and disposition thereof. Table F presents a list of cellular stress response pathways along with the key regulatory transcription factors, cellular biosensors, and some known responsive gene targets.

| **TABLE F. Cellular Stress Response Pathways** | | | |
|---|---|---|---|
| **Pathway** | **Transcription Factor** | **Sensor** | **Representative Gene Targets** |
| Heat Shock | HSF1 | Hsp90 | Hsp27, Hsp70, Hsp90 |
| Hypoxia | HIF1 | VHL | VEGF, PDK1, GLUT1 |
| ER Stress | XBP1, ATF4, ATF6 | BiP/Grp78 | BiP, CHOP, p58 |
| DNA Damage | P53 | MDM2 | P21, GADD45, BAX, RAD51 |
| Oxidative Stress | Nrf2 | Keap1 | HMOX1, PRDX1, GSTA2, p66Shc |
| Inflammation | NF κ B | I κ B | COX2, iNOS, ICAM, IL-6, IL-8 |

### (i) Heatshock

Induced by exposure to elevated temperatures, heavy metals, toxins, oxidants, and bacterial and viral infections, the heat shock response is characterized by increased expression of heat shock proteins (Hsps). Heat shock proteins ensure survival under stressful conditions, but if the stress is too severe, a signal that leads to programmed cell death is activated. Hsps play essential roles in the synthesis, transport, and folding of proteins and are often referred to as molecular chaperones. Exemplary cell stress related proteins involved in cellular heat shock response are detailed below.

*HSF1:* In eukaryotic cells, the heat shock response involves transcriptional activation mediated by a transcription factor known as heat shock factor (HSF). Several members of the HSF family have been found in vertebrates (murine and human HSF1, 2, and 4 and a unique avian HSF3) and plants. HSF1, an HSF prototype, and HSF3 are responsible for heat-induced Hsp expression. In unstressed cells, HSF1 is present in both the cytoplasm and nucleus in a monomeric form that has no DNA binding activity. When cells are stressed, HSF1 homotrimerizes, acquires DNA-binding activity, translocates from the cytoplasm to the nucleus, is hyperphosphorylated, and becomes transcriptionally competent. Once activated, HSF1 binds to the heat shock element (HSE), a specific DNA recognition sequence located in the heat shock responsive genes within minutes of temperature elevation.

*HSP70:* The mechanism of stress activation of HSF1 may be twofold. First, stress induces protein unfolding, which results in accumulation of nonnative protein. Nonnative protein appears to be the common proximal inducer of HSF1 activity and enhanced hsp gene expression in the stressed cell. Second, an Hsp, possibly Hsp70, may play a role in retaining HSF1 in the inactive state in the absence of stress and/or in returning the factor to this state following a stressful event, in that Hsp70 may function as a repressor of HSF1 activation.

The expression of the inducible Hsp70 has been shown to enhance the survival of mammalian cells exposed to numerous types of stimuli that induce stress and apoptosis. Hsp70 members are abundantly expressed in many malignant human tumors. Molecular chaperones such as members of the hsp70 family may be important in autoregulation of the heat shock response as they facilitate protein folding by stabilizing intermediate folded states of nascent proteins, thus preventing them from engaging in inappropriate interaction that may lead to irreversible, nonspecific aggregation. Further, Hsp70 has been shown to contribute to protection of myocardium from ischemic injuries. The basis for the cardioprotective activity of Hsp70 is likely to be related to its ability to prevent protein aggregation during ischemic stress.

*HSP27:* Hsp27 is a member of the family of small heat shock proteins (sHsps). Their common trait is the conserved α-crystallin domain, which refers to the most prominent family member, the eye-lens protein α-crystallin. Functionally, sHsps are ATP-independent chaperones that interact with large numbers of partially folded target proteins to prevent their aggregation upon stress-induced unfolding. It is currently held that sHsps serve as a storage depot for unfolded proteins, which can be refolded in the presence of other larger chaperones such as the Hsp70 and Hsp100 proteins. It seems that sHsps are not only able to form soluble complexes with their unfolding clients but sometimes, especially when protein unfolding is massive in the cell, they are sequestered into the aggregates. Additional functions specific for Hsp27 include inhibition of apoptosis (blocks the activation of procaspase-9: interacts with the outer mitochondrial membrane and interferes with the activation of cytochrome c/Apaf-1), protection of cytoskeletal structure, and activation of proteasome (i.e., binds to ubiquitinated proteins and to the 26S proteasome).

### (ii) Hypoxic stress

The oxygen homeostasis control occurs at the level of an entire organism as well as at the level of a single cell. Intracellular levels of oxygen have to be maintained within tight limits because of the balance between the need for O₂ in many metabolic processes and the high toxicity of O₂ beyond certain level. At the cellular level, decreased O₂ tension (hypoxia) leads to the activation of alternative metabolic pathways that do not require molecular oxygen. The switch from aerobic metabolism to anaerobic glycolysis is mediated by the induction ofglycolytic enzymes and expression of glucose transporters. Additionally, the expression of various stress proteins responsible for cell death or survival is upregulated. Further adaptations that occur at the tissue and systemic levels lead to the increase in O₂ delivery. They include induction of erythropoiesis (red blood cell production), angiogenesis (new vessel formation), and hyperventilation. Among the various upregulated proteins there is erythropoietin (EPO), the main growth factor inducing maturation of erythrocytes, and vascular endothelial growth factor (VEGF), the major mediator of angiogenesis and vascular permeability. The hypoxia-dependent regulation of these and other proteins occurs at the transcriptional level and is mediated by hypoxia-inducible transcription factor (HIF-1). The consensus DNA sequence for HIF-1 binding is common for many genes upregulated by low oxygen tension. Exemplary cell stress related proteins involved in cellular hypoxic stress response are detailed below.

*HIF-1α:* Hypoxia-inducible factor-1 (HIF1) is a transcriptional activator that functions as a key regulator of cellular and systemic oxygen homeostasis. HIF-1 is induced not only in response to reduced oxygen availability but also by other stimulants, such as nitric oxide, various growth factors, or direct inhibitors of prolyl and asparaginyl hydroxylases. Therefore, it seems to be a crucial transcription factor elicited by a wide range of stresses such as impaired oxygenation, inflammation, energy deprivation, or intensive proliferation. HIF-1 also plays significant roles in cancer progression as well as in cardiovascular diseases.

HIF-1 is a heterodimer composed of α and β subunits. The regulation of HIF-1activity concerns mostly the α subunit (HIF-1α) and occurs at multiple levels such as protein stabilization, post-translational modifications (prolyl and asparaginyl hydroxylation), nuclear translocation, dimerization, transcriptional activation, and interaction with other proteins. Moreover, changes in mRNA expression and alternative splicing of both subunits have been observed. Under normoxic conditions, however, when HIF-1α and HIF-1β are constitutively transcribed and translated, the abrogation of HIF-1 activity results mainly from constitutive HIF-1α degradation.

The C-terminal part of the HIF-1α protein contains an oxygen-dependent degradation domain (ODD) responsible for degradation of HIF-1α under normoxic conditions. The HIF-1α half-life under normoxic conditions is less than 10 minutes and the protein is hardly detectable. HIF-1 activation, or HIF-1α stabilization, is induced by the lack of molecular oxygen.

Cells comprising HIF-1α knock-in with various reporter systems may, therefore, be able to detect the altered HIF-1α stabilization, post-translational modifications, nuclear translocation, dimerization, transcriptional activation, and interaction with other proteins when the cell is contacted with a stimulus that may induce hypoxic stress. Cells comprising HIF-1α knock-in with various reporter systems may be used to profile drugs for its effect on cellular oxygen homeostasis.

*Pyruvate dehydrogenase kinase 1 (PDK1):* PDK1 is a mitochondrial enzyme that inhibits pyruvate dehydrogenase via phosphorylation. Inactivation of respiratory chain proteins is a key cellular response to oxygen deprivation. A key check point in the metabolism of glucose occurs at pyruvate, which can be converted either into lactate or into acetyl CoA by PDK1. The PDK1 gene is induced during hypoxia in an HIF-1α-dependent manner, thus leading to pyruvate's shunting away from the mitochondria and, in turn, to reducing activity of the TCA cycle. This mechanism is used by the cell to maintain the intracellular oxygen concentration.

*Glucose Transporter 1 (GLUT1):* Under hypoxia, cells dramatically increase anaerobic glycolysis by up-regulating the expression and the activity of glycolytic enzymes and increasing glucose transport at the membrane level. Glucose transport at the membrane is largely achieved by GLUT-1, a member of the family of facilitative Na+-independent transporters. GLUT-1 is found in diverse tissues and cell types and functions to provide adequate energy supply under hypoxia despite the reduction of oxidative phosphorylation. GLUT1 mRNA and transporter expression are rapidly and markedly up-regulated in cells in response to hypoxic stress.

*Vascular Endothelial Growth Factor (VEGF):* At the cellular level, hypoxia induces expression of several angiogenic genes, most notably vascular endothelial growth factor (VEGF). Although multiple factors can influence the expression of VEGF, during hypoxia VEGF expression is directly controlled by the transcription factor HIF-1α.

### (iii) ER stress

In eukaryotic cells, the endoplasmic reticulum (ER) is the organelle for the folding and processing of proteins destined for secretion, the plasma membrane, or secretory organelles. These proteins are translocated into the ER lumen where they are modified and oligomerize to acquire their correct folding structure. The high demand for efficient protein folding and secretion processes in these cells constitutes a constant source of stress initiated by the presence of large amounts of misfolded proteins that are normally generated during the protein maturation process. These folding subproducts are eliminated through ER-associated degradation (ERAD), where misfolded proteins translocate to the cytosol and are degraded by the proteasome.

ER stress is also triggered by conditions that alter proteostasis associated with perturbations in protein maturation, expression of certain mutant proteins, decreased chaperone function, abnormal ER calcium content redox metabolism, altered trafficking, and many others. Endoplasmic reticulum (ER) stress is a hallmark feature of secretory cells and many diseases, including cancer, neurodegeneration, diabetes and inflammation. As an initial response to ER stress, cells activate the unfolded protein response (UPR) to decrease the unfolded protein load and recover homeostasis. If this cannot be achieved or the repair process is overwhelmed, programmed cell death (apoptosis) will be initiated.

The UPR functions through three distinct stress sensors located at the ER membrane: IRE1α (inositol-requiring transmembrane kinase/endonuclease), ATF6 (activating transcription factor 6), and PERK (PKR-like ER kinase). Through these sensors, UPR regulates genes involved in protein entry into the ER, folding, glycosylation, ERAD, protein quality control, redox metabolism, autophagy, lipid biogenesis, and vesicular trafficking. The ER stress response contains at least three components: (1) transcriptional induction of ER chaperones and folding enzymes, such as BiP/GRP78, GRP94, and protein disulfide isomerase, (2) translational attenuation to prevent the further loading of proteins into the ER, and (3) ER-associated degradation
(ERAD) to clear misfolded proteins out of the ER. Exemplary cell stress related proteins involved in cellular ER stress response are detailed below.

*BiP*/*Grp78:* The ER chaperone BiP has been used extensively as an indicator of the onset of the UPR. ATF6 and its essential coactivator YY1, a constitutively expressed multifunctional transcription factor, activate the BiP/Grp78 promoter only under ER stress conditions. BiP represses PERK and IRE1 activation by keeping them in their monomeric inactive states, and dissociation of BiP during ER stress correlates with IRE1 and PERK activation. ATF6 exists in a complex with BiP in unstressed cells and dissociates upon ER stress. Loss of BiP binding during ER stress relieves the inhibition to ATF6 activity, allowing ATF6 to be transported to the Golgi. Overexpression of BiP attenuates ATF6 activation. Therefore, this is an autoregulatory model by which ER chaperones control their own expression..

*PERK:* PERK is an ER transmembrane eIF2α protein kinase that mediates the ER-stress induced translational attenuation. eIF2α is a translation initiation factor. Activated PERK phosphorylates eIF2α, inhibiting protein translation into the ER and alleviating ER stress by decreasing the overload of misfolded proteins. PERK also contributes to transcriptional induction by promoting the selective translation of the transcription factor ATF4 which activates the ER stress target gene CHOP/GADD153. PERK knockout would create a cell line that is more sensitive to ER stress-induced apoptosis, and such a system may be used for screening compounds that induce ER stress.

*IRE1:* Transcriptional induction is mediated by IRE1. IRE1 is an ER transmembrane protein with cytoplasmic protein kinase and endoribonuclease domains. Little is known about the regulation of IRE1 activity. The mechanism of IRE1 activation by ER stress appears to involve the binding of BiP to its luminal domains. This inhibits IRE1 signaling by maintaining it in its monomeric states. ER stress then causes the dissociation of BiP, allowing the oligomerization and activation of IRE1. The transcription factor XBP1 was identified as a mammalian target of IRE1 splicing.

*A TF4 and A TF6:* ATF4 (activating transcription factor 4) is a transcription factor that upregulates UPR genes that function in amino acid and redox metabolism, including chop/gadd153 and gadd34. When under ER stress, activated PERK phosphorylates and inhibits translation initiator factor eIF2α, decreasing the synthesis of proteins and the overload of misfolded proteins at the ER. Phosphorylation of eIF2α allows the expression of ATF4. A second UPR pathway is initiated by ATF6α and ATF6β, type II ER transmembrane proteins whose cytosolic domain encodes a bZIP transcriptional factor. Upon ER stress induction, ATF6 is processed at the Golgi, releasing its cytoplasmic domain, which acts as a transcriptional activator controlling many UPR genes related to ERAD and folding at the ER among others. ATF6 binds to the ER stress response element (ERSE) in the promoters of ER stress target genes such as BiP and GRP94. ATF6 binds to the ERSE only in the presence of the CCAAT box binding factor NF-Y/CBF.

*XBP1:* A third UPR pathway involves XBP1. In cells undergoing ER stress, IRE1a autophosphorylates, leading to the activation of its endoribonuclease domain. This activity mediates the splicing of the mRNA encoding XBP1, which is a transcriptional factor that upregulates many essential UPR genes involved in folding and protein quality control and regulates ER/Golgi biogenesis. Spliced XBP1 (XBP1s) controls the upregulation of a broad spectrum of UPR-related genes involved in protein folding, protein entry to the ER, ERAD, and protein quality control. Other XBP-1 regulated genes are linked to cell differentiation, signaling, and DNA-damage pathways, trafficking pathways, transcriptional regulatory networks, modulators of lipid and ion composition, and vacuolar function. In still another embodiment, the cell stress protein with disrupted expression may be XBP1.

### (iv) DNA damage

Genetic stability requires that any damage sustained by the genome be repaired before the cell divides. Checkpoint signals resulting from the detection of DNA damage arrest the cell cycle to allow time for the cell's repair systems to act, with apoptosis as an alternative outcome. Chromosomal abnormalities such as gene amplifications, translocations, inversions and deletions are often seen in tumor cells, suggesting that karyotype instability is involved in tumorigenesis. Common to the occurrence of these chromosomal defects is the formation and rejoining of DNA strand breaks. Double strand breaks (DSBs) in DNA are an especially hazardous lesion caused by ionizing radiation and are repaired by two major repair pathways: non homologous end-joining (NHEJ) and homologous recombination (HR). The fidelity of resolution of a break produced by a DNA damaging agent, viral integration or normal cellular events such as recombination determines whether the wild type genome is restored. Thus, it is critical that the processes that repair DNA strand breaks are strictly regulated. Exemplary cell stress related proteins involved in cellular DNA damage stress response are detailed below.

*p53:* TP53 encodes the p53 tumor suppressor protein, p53. p53 is a multifunctional molecule that influences the cell cycle, DNA repair, suppresses aberrant recombination, and apoptosis by regulating transcription and interacting directly with other proteins. These functions enable p53 to contribute to the maintenance of genomic stability in the presence of a mutagenic environment. Following DNA damage, p53 upregulates the expression of p21Waf1 protein to effect a G1 cell cycle arrest and G2 arrest in order to prevent the replication of damaged DNA. p53 also binds to and modulates the DNA repair activity of the nucleotide excision repair factors XPB and XPD, influencing the repair machinery directly. If these cell cycle arrest and DNA repair functions fail to restore the genome to a wild-type state, p53 may also direct the elimination of the damaged cell via apoptosis.

Approximately 50% of all cancers have mutations in TP53. One of the main functions of mutant p53 is to up-regulate RAD51. In addition to the loss of cell cycle control, the lack of wild type p53 function also relates to chromosomal abnormalities. Gene amplification is elevated in the absence of p53 function. Homologous recombination is increased in human fibroblasts with inactivated p53. p53 may also influence recombination directly. The p53 C-terminal basic domain can direct both DNA single strand annealing and strand transfer activities, events that occur during recombination.

*ATM and ATR:* Cell cycle checkpoints are regulatory pathways that govern the order and timing of cell cycle transitions to ensure completion of one cellular event prior to commencement of another. The key regulators of the checkpoint pathways in the mammalian DNA damage response are the ATM (ataxia telangiectasia, mutated) and ATR (ATM and Rad3-related) protein kinases. Both of these proteins belong to a structurally unique family of serine-threonine kinases characterized by a C terminal catalytic motif containing a phosphatidylinositol 3-kinase domain. ATM is the primary mediator of the response to DNA double strand breaks (DSBs) that can arise by exposure to ionizing radiation (IR). ATR, on the other hand, plays only a back-up role in
the DSB response, but directs the principle response to UV damage and stalls in DNA replication.

Once activated, ATM and ATR phosphorylate an overlapping set of DNA repair/checkpoint targets. For ATM these include Gadd45, p53, replication protein A, Chk2, NBS1, BRCA1, and c-Abl, consistent with it being a major regulator of the cell cycle reaction to genome damage. Targets for ATR include p53 and CHK1. One important effect of these phosphorylation events is the control of cell-cycle checkpoints. Depending on the nature of the DNA lesion, both ATM and ATR can induce the G1/S, G2/M and S-phase checkpoints. By doing so, they ensure that the cell accurately repairs the DNA damage before DNA replication or cell division occurs.

In the case of ATR, the ability to phosphorylate downstream substrates requires an associated protein (ATRIP). ATM is ubiquitously expressed, although at heterogeneous levels in different cell and tissue types. A fraction of ATM is also present in the cytoplasm. However, ATM is present predominantly within the nucleus of cultured human cells, which is consistent with the proposed role of ATM in cellular response to DNA damage.

In the thymus, p53 is phosphorylated directly by nuclear ATM after IR (ionizing radiation), leading to transcriptional activation of p21Waf1/Cip1 and consequential cell-cycle arrest. In the absence of ATM, this pathway is disrupted, and this defect perhaps results in the immunodeficiency and abnormal cellular responses to IR seen in patients with AT (ataxia telangiectasia), an autosomal recessive disorder. Genetic instability and abnormalities of the nervous, immune and reproductive systems are among the complex clinical features of AT, which also include a predisposition to lymphoid malignancy and extreme radiosensitivity.

*RAD51:* Rad51, originally described in yeast, is closely related to the Escherichia coli RecA protein that catalyzes DNA strand transfer and recombination. Yeast Rad51 mutants fail to repair double strand breaks, exhibit spontaneous and radiation induced chromosomal loss, and harbor a defect in both mitotic and meiotic recombination. Furthermore, yeast Rad51 appears to be required for conversion of double strand breaks to recombination intermediates, and localizes to presumptive foci of recombination in yeast meiosis. The human homologue of yeast Rad51 shares 83% sequence homology with the yeast protein, and share a common central DNA binding domain with RecA. Human Rad 51 (hRad51) is nuclear localized, forms nucleoprotein filaments structurally similar to those formed by yeast Rad51, underwinds DNA in these filaments, possesses a DNA dependent ATPase activity, and promotes ATP-dependent homologous pairing and strand transfer reactions. Both proteins mediate sister chromatid exchange (SCE), which reflects the post-replicational repair of spontaneous DNA damage by recombination with the intact sister chromatid.

RAD51 is overexpressed in many tumors. The wild-type p53 protein plays an important role both in suppressing the transcriptional expression of RAD51 and in down-regulating RAD51 protein activity. The high levels of RAD51 are involved in tumor progression by destabilizing the genome. Elevated RAD51 may also confer a DNA replication advantage during the more rapid cell divisions that follow the activation of oncogenes and inactivation of tumor suppressors.

*GADD45:* GADD45 (growth arrest and DNA damage-45) gene encodes the growth arrest and DNA damage-45 protein, a known transcriptional target of BRCA1. GADD45 is upregulated by Vpr in an ATR-dependent manner following stressful growth arrest conditions and treatment with DNA-damaging agents. The protein encoded by this gene responds to environmental stresses by mediating activation of the p38/JNK pathway via MTK1/MEKK4 kinase. The DNA damage induced transcription of this gene is mediated by both p53-dependent and -independent mechanisms.

### (v) Oxidative stress

Reactive oxygen species (ROS) are generated during aerobic respiration and normal metabolic processes, and they are also byproducts of metabolism of a wide range of environmental agents. Production of high levels of reactive oxygen (ROS) and nitrogen species (RNS) is a condition that generally leads to as oxidative stress. High levels of ROS are detrimental to the cell, since they react readily with intracellular molecules, causing cell injury and death. Oxidative stress may also play a role in carcinogenesis through an unbalanced generation of reactive oxygen species that leads to genetic instability.

The induction of cytoprotective antioxidant enzymes in response to oxidative stress is critical for the maintenance of homeostasis of cellular redox. Cells are equipped with a variety of defense mechanisms that work in parallel or in sequence to minimize ROS levels. These defenses include enzymes that are involved in ROS metabolism and biotransformation of xenobiotics. The induction of these enzymes is regulated primarily at the transcriptional level. Examples of these enzymes include superoxide dismutases, glutathione peroxidases, thioredoxins, and heme-oxygenases as well as phase 2 enzymes, such as glutathione S-transferases. In addition to enzymatic defenses, cells are also equipped with molecules such as glutathione, metallothioneins, and ferritins that scavenge ROS and metal ions. Exemplary cell stress related proteins involved in cellular oxidative stress response are detailed below.

*Nrf2:* A major mechanism in the cellular defense against oxidative stress is activation of the Nrf2-ARE (nuclear factor E2-related factor 2-antioxidant response element) signaling pathway, which controls the expression of genes whose protein products are involved in the detoxification and elimination of reactive oxidants. Potent inducers of ARE activity include H₂O₂, chemical compounds undergoing redox cycling or being metabolically transformed to a reactive or electrophilic intermediate, and compounds reacting with sulfhydryl groups such as diethyl maleate, isothiocyanates, and dithiothiones.

Nrf2 is a transcription factor and regulates cellular redox status by controlling both the inducible and constitutive gene expression mediated by the ARE. Genetic disruption of Nrf2 decreases levels of both the basal and inducible expression of several critical antioxidant enzymes. Some of the critical enzymes include: glutamate-cysteine ligase catalytic subunit (Gclc), glutamate cysteine ligase modifier subunit (Gclm), glutathione peroxidase 2 (Gpx2), and hemeoxygenase 1 (Hmox1).

Activation of the Nrf2-ARE pathway provides protection from glutamate- and H202-induced cell death. The Nrf2-mediated response alters susceptibility to carcinogenesis, acute chemical toxicity, oxidative stress, asthma, acute inflammation, septic shock and neurodegenerative diseases such as Alzheimer's disease and Parkinson's disease. Studies using natural and synthetic chemical inducers that activate Nrf2 signaling have demonstrated protective efficacy in many animal models of disease. Conversely, studies in Nrf2-disrupted mice indicate they exhibit increased sensitivity to many of these diseases. Thus, activation of Nrf2-ARE signaling constitutes a broad protective response, making Nrf2 and its interacting partners important targets for chemoprevention.

*HMOX1:* HMOX1 (heme oxygenase (decycling) 1) is a human gene that encodes for the enzyme heme oxygenase 1 (HMOX1). Heme oxygenase cleaves heme to form biliverdin in heme catabolism. Heme oxygenase activity is induced by its substrate heme and by various nonheme substances. Heme oxygenase has two isozymes, an inducible heme oxygenase-1 (HMOX1) and a constitutive heme oxygenase-2 (HMOX2). HMOX1 is highly expressed in various solid tumors, and has an important role in rapid tumor growth.

*PRDX1:* PRDX1 (peroxiredoxin 1) gene encodes a member of the peroxiredoxin family of antioxidant enzymes, which reduce hydrogen peroxide and alkyl hydroperoxides. The encoded protein PRDX1 plays an antioxidant protective role in cells in redox regulation, and may have a proliferative effect and play a role in cancer development or progression. PRDX1 may participate in the signaling cascades of growth factors and tumor necrosis factor-alpha by regulating the intracellular concentrations of H₂O₂.

*GSTA2:* GSTA2 (glutathione S-transferase A2) is a major detoxification enzyme encoded by the GSTA2 gene. Its response to oxidative stress is regulated at the transcriptional level mediated by ARE in its promoter. The glutathione S-transferase (GST) supergene family comprises gene families that encode isoenzymes that are widely expressed in mammalian tissue cytosols and membranes. GST catalyse the conjugation of reduced glutathione (GSH) with a wide variety of electrophiles and various compounds, such as carcinogens, therapeutic drugs, environmental toxins, and oxidized DNA or lipid resulted from oxidative stress.

The genes encoding these enzymes are known to be highly polymorphic. These genetic variations can change an individual's susceptibility to carcinogens and toxins as well as affect the toxicity and efficacy of some drugs. At present, eight distinct classes of the soluble cytoplasmic mammalian glutathione S transferases have been identified: alpha, kappa, mu, omega, pi, sigma, theta and zeta. GSTA2 gene encodes a glutathione S-tranferase belonging to the alpha class. The alpha class genes, located in a cluster mapped to chromosome 6, are the most abundantly expressed glutathione S-transferases in liver. In addition to metabolizing bilirubin and certain anti-cancer drugs in the liver, the alpha class of these enzymes exhibit glutathione peroxidase activity thereby protecting the cells from reactive oxygen species and the products of peroxidation.

*Keap1:* Keap1 (Kelch-like ECH-associated protein 1) affects the accumulation of Nrf2 in the cell. Nrf2 has been found to be a highly unstable protein subjecting to proteolytic degradation, and is with a half-life time of about 15 minutes. In non-stressed cells, the Nrf2 protein level is low. Keap1 appears to promote Nrf2 ubiquitylation in a constitutive manner. Upon interaction with Keap1, Nrf2 is targeted directly for ubiquitylation and degradation. When there are stress signals, the Keap1-Nrf2 association is disrupted, either through Keap1 nucleocytoplasmic shuttling or Nrf2 translocation, Nrf2 is stabilized and effects its transcriptional activity in the nucleus.

Keap1 has a large number of Cys residues within its primary structure, which makes it possible to conduct inhibitory modulation of its activity and thus lead to Nrf2 activation by thiol-reactive chemical species. A number of reactive Cys residues include those at positions 257, 273, 288, and 297 in Keap1 are putative sites important for regulation of its interaction with Nrf2. In still another embodiment, the cell stress protein with disrupted expression may be Keap1.

*p66Shc:* The p66Shc (SHC (Src homology 2 domain-containing) transforming protein 1) gene, which encodes a protein belonging to a family of adaptors for signal transduction in mitogenic and apoptotic responses. Normally, p66Shc is tyrosine phosphorylated (activated) by various extracellular signals including EGF and insulin. However, serine phosphorylation of p66Shc can occur after oxidative stress either in association with or independently of tyrosine phosphorylation. p66Shc serine phosphorylation has been linked to inactivation of members of forkhead transcription factors, resulting in increased intracellular oxidant levels and increased sensitivity to apoptosis. Knocking out p66Shc allows moderately elevated activity of forkhead transcription factors and better-equipped antioxidant defenses at the cellular level. In addition, methylation of the p66Shc promoter has important implications in its expression regulation, which may contribute to variations of longevity among individuals. Genetic variation or p66Shc knockout may create a cell line with altered sensitivity to oxidative stress, which may be used for screening compounds that induce oxidative stress.

### (vi) Inflammation

Inflammation is a protective response by an organism to limit tissue injury and to initiate the healing response. Nuclear factor kappa B (NFκB) is the key transcription factor that regulates the inflammatory response. In most cells, NFκB is held in a latent state in the cytoplasm by interaction with IκB, an inhibitory sensor. Pro inflammatory signals destabilize the IκB/NFκB complexes and allow translocation of NFκB to the nucleus where it triggers the expression of numerous target genes. Gene targets include inflammatory genes (e.g. COX2, iNOS), adhesion molecules (ICAM, VCAM), cytokines (IL-1, IL-6, TNFα), chemokines (IL-8, MCP-1) and other stress response genes. Inflammation is also known to down-regulate many drug metabolizing enzymes.

### (e) Single Nucleotide Polymorphisms in ADME/Tox Genes

Drug response is a complex phenomenon involving the interaction of several different components including drug metabolizing enzymes, drug transporters, drug targets and regulatory factors. Drug response can differ greatly amongst individuals, and genetic variation plays an important role in determining a drug's absorption, distribution, metabolism and interaction with its target. Single nucleotide polymorphisms (SNPs) are the most common form of human genetic variation. The SNPs may be in a coding region or a non-coding region, some SNPs may change a gene's expression level, temporal or regional expression specificity, or even gain-of-function or loss-of function at its protein level. Some SNPs, however, may not be functionally significant despite the clinic importance of SNPs in general.

Genes for drug transporters, drug metabolizing enzymes, regulatory nuclear receptors and stress response pathways all have SNPs, although they may be at a varied frequency. The genetic variances may confer different substrate specificities to various drugs, may affect drug absorption and distribution, with the potential of altering drug effectiveness in large populations. Many of these SNPs may be of clinical significance.

The large number of identified SNPs within the transporters, drug metabolizing enzymes, nuclear receptors, and stress response pathways disclosed herein make it impractical to list them in a meaningful way. However, as an example of how an individual SNP might be selected and modeled in an appropriate cell line, Table G presents exemplary SNPs identified in human CYPs, including their relative frequency and known functional effects. For example, in one embodiment the chosen SNP might be CYP2D6*10 which may be created in an appropriate hepatocyte cell line.

| **TABLE G. Key Polymorphisms in Human CYPs** | | | |
|---|---|---|---|
| **Enzyme** | **Polymorphism Frequency** | **Functional Effects** | **Most Important Variants** |
| CYP1A1 | Relatively high | Unproven | None |
| CYP1A2 | High | Rare | *CYP1A2*F, CYP1A2*K* |
| CYP1B1 | Rare null alleles; frequent missense | At least 7 haplotypes with similar activity | *CYP1B1*7* |
| CYP2A6 | High in Orientals, less frequent in Caucasians | Important for metabolism | *CYP2A6*1B, CYP2A6*4, CYP2A6*9, CYP2A6*12* |
| CYP2B6 | High | Reduced drug metabol ism | *CYP286*5 , CYP286*6* |
| CYP2C8 | High | Reduced drug metabolism | *CYP2C8*3* |
| CYP2C9 | Relatively rare in Caucasians | Very significant | *CYP2C9*2,* |
| CYP2C19 | High | Very significant | *CYP2C19*2, CYP2C19*3, CYP2C19*17* |
| CYP2D6 | Very high | Very significant | *CYP2D6*2xn, CYP2D6* 4, CYP2D6*5,* |
| CYP2E1 | Low | No significant cases have been | None |
| CYP3A4 | Low | No or small | *CYP3A4*1B* |
| CYP3A5 | High | Significant | *CYP3A5*3, CYP3A5*6* |
| CYP3A7 | Low | Some | *CYP3A7*2* |

| | | | |
|---|---|---|---|
| From: Johansson I and Ingelman-Dundberg M, Genetic polymorphism and toxicology- with emphasis on cytochrome p450, Toxico/ Sci 120, 1-13 (2011) | | | |

### (f) Optional Expression of Reporter Protein(s)

In certain embodiments, the cell having disrupted expression of a ADME/Tox protein encoded by ABCG2 may further comprise expression of at least one reporter protein. Thus, the cell may also be engineered to express at least one reporter protein. Stated another way, the cell may be termed a "knock-in" with regards to expression of the reporter protein(s), as well as having disrupted expression of a ADME/Tox protein encoded by ABCG2.

Suitable reporter proteins include, without limit, fluorescent proteins, luciferase and variants thereof, epitope tags (such as, e.g., HA, FLAG, 6xHis, etc.), glutathione-S-transferase (GST), beta-galactosidase, maltose-binding protein
(MBP), cloramphenicol acetyltransferase (CAT), and neomycin phosphotransferase (neo). Non limiting examples of suitable fluorescent proteins include green fluorescent proteins (e.g., GFP, GFP-2, tagGFP, turboGFP, EGFP, Emerald, Azami Green, Monomeric Azami Green, CopGFP, AceGFP, ZsGreen1), yellow fluorescent proteins (e.g. YFP, EYFP, Citrine, Venus, YPet, PhiYFP, ZsYellow1,), blue fluorescent proteins (e.g. EBFP, EBFP2, Azurite, mKalama1, GFPuv, Sapphire, T-sapphire,), cyan fluorescent proteins (e.g. ECFP, Cerulean, CyPet, AmCyan1, Midoriishi-Cyan), red fluorescent proteins (mKate, mKate2, mPlum, DsRed monomer, mCherry, mRFP1, DsRed-Express, DsRed2, DsRed-Monomer, HcRed-Tandem, HcRed1, AsRed2, eqFP611, mRasberry, mStrawberry, Jred), and orange fluorescent proteins (mOrange, mKO, Kusabira-Orange, Monomeric Kusabira-Orange, mTangerine, tdTomato). Preferred reporter proteins include GFP and other fluorescent proteins and luciferases.

In some embodiments, the reporter protein may be expressed from a chromosomally integrated nucleic acid sequence. A nucleic acid encoding the reporter protein may be integrated randomly in the genome of the cell using well known procedures. Alternatively, a nucleic acid encoding the reporter protein may be integrated in a targeted location in the genome of the cell using targeting endonuclease technology described below section (II)(a). The chromosomally integrated nucleic acid sequence encoding the reporter protein may be operably linked to a transcriptional control element this is endogenous to the cell or it may be operably linked to an exogenous transcriptional control element (such as a constitutive viral promoter, for example).

In other embodiments, the reporter protein may be expressed from an extrachromosomal nucleic acid sequence. For example, a nucleic acid encoding the reporter protein may be within a vector that remains extrachromosomal. Suitable vectors are well known in the art, as are means for introducing such vectors into the cell.

### (g) Cell Types

The type of cell comprising disrupted expression of a ADME/Tox protein encoded by ACBG2, as detailed above, is a human cell. In some instances, the cell may be a primary cell, a cultured cell, a stem cell, or an immortalized cell line. The cell may be of a variety of cell types, e.g., intestinal cells, liver cells, kidney cells, neuronal cells, muscle cells, lung cells, cervical cells, bone cells, skin cells, fibroblasts, myoblasts, T or B cells, macrophages, epithelial cells, and so forth. Alternatively, the cell may be a stem cell. Suitable stem cells include progenitor cells, adult stem cells, pluripotent stem cells, induced pluripotent stem cells, multipotent stem cells, oligopotent stem cells, and unipotent stem cells.

The cell line may be any established cell line or a primary cell line that is not yet described. In the case of primary cell lines, these cells may be modified to enhance their ability to propagate and continue dividing in cell culture. This may involve targeting pathways known to regulate cellular senescence, for example p16, p53, pRb, etc. For an extensive list of mammalian cell lines, those of ordinary skill in the art may refer to the American Type Culture Collection catalog (ATCC®, Mamassas, VA).

Exemplary cells are human cells or humanized cells. In particular, exemplary human cells may be intestinal cells or intestinal cell lines, hepatic cells or hepatic cell line cells, kidney cells or kidney cell line cells, skin cells or skin cell lines.

In one embodiment, the intestinal cell may be a human cell chosen from Caco-2, C2BBe1, COLO-205, COLO-320DM, COLO-60H, COLO-94H, CX-1, CX- 2, HRT-18, HT-29, HUTU-80, LOVO, LS-174T, LS-513, SW-1116, SW-403, SW-480, SW-707, SW-948, T84, WiDr, IEC-18, IEC-6, and derivative cells thereof. An exemplary human intestinal cell may be a Caco-2 cell line cells or derivatives thereof, such as C2BBe1 cells.

In another embodiment, the hepatic cell may be a human cell chosen from HepaRG, HEP-G2, Hep-G2-C3A, Hep 38, SK-HEP-1, Chang-Liver, PLC PRF-5, THLE-2, Huh7, HLE, Fa2N-4, HC-04, and derivative cells thereof. An exemplary hepatic cell may be the HepaRG cell line cell or a derivative thereof. An advantage of HepaRG cells, for example, is that many phase I drug metabolism enzymes are expressed. For example, CYP1A2, CYP2A6, CYP2B6, CYP2C8, CYP2C9, CYP2C19, CYP2D6, CYP2E1 and CYP3A4 are expressed in HepaRG cells.

In yet another embodiment, the kidney cell may be a human cell chosen from ciPTEC, 293 (HEK-293), HK-2, and derivative cells thereof. An exemplary kidney cell may be a ciPTEC cell or derivative thereof.

In preferred embodiments, the cells are of a type that is widely used for studying drug absorption, disposition and/or metabolism, such as intestine, liver, kidney, and brain. In an exemplary embodiment, the cells may be Caco-2 cells or derivatives thereof. In another exemplary embodiment, the cells may be HepaRG cells or derivatives thereof. In yet another exemplary embodiment, the cells may be ciPTEC cells or derivatives thereof.

### (h) Preferred Embodiments

The cell may be a human cell line comprising disrupted expression of BCRP (coded by ABCG2). Also discussed is a human cell line comprising disrupted expression of MDR1 (coded by ABC1). In another preferred embodiment, the cell may be a human cell line comprising disrupted expression of both BCRP and MDR1. In an exemplary embodiment, the cells detailed above comprises inactivated chromosomal sequences encoding BCRP and/or MDR1 such that expression of BCRP and/or MDR1 is substantially eliminated (i.e., knocked out). Each of the preferred cell lines may further comprise "knocked-in" expression of at least one reporter protein.

### (II) Methods for Preparing Cells Having Disrupted Expression of ADME/Tox Protein(s)

Yet another aspect of the present disclosure provides a method for preparing a cell having disrupted expression of a ADME/Tox protein encoded by ABCG2 according to claim 2. Expression may be disrupted by editing the chromosomal sequence
of ABCG2 encoding the ADME/Tox protein using targeting endonucleases.

In general, the method comprises introducing into the cell at least one agent that modifies the expression of ADME/Tox protein encoded by ABCG2. Suitable cells are detailed above in section (I)(f).

### (a) Targeting Endonuclease Mediated Genome Editing

Expression of the ADME/Tox protein encoded by ABCG2 is disrupted at the level of DNA via targeted genome editing as mediated by targeting endonucleases. A targeting endonuclease is an entity that recognizes and binds a specific double-stranded chromosomal DNA sequence and introduces a double stranded break at a targeted cleavage site in the chromosomal sequence. As used herein, "genome editing" or "chromosomal editing" refers to the modification or editing of a genomic or chromosomal sequence such that no protein product is made, a reduced level of protein product is made, or an altered version of the protein produce is made. The edited chromosomal sequence may comprise a deletion of at least one nucleotide, an insertion of at least one nucleotide, or a substitution of at least one nucleotide.

In general, methods for targeted genome editing comprise introducing into a cell at least one targeting endonuclease or nucleic acid encoding a targeting endonuclease that recognizes a target sequence in the chromosomal sequence and is able to cleave a site in the chromosomal sequence, and, optionally, (i) at least one donor polynucleotide comprising a sequence for integration, the sequence flanked by an upstream sequence and a downstream sequence that share substantial sequence identity with either side of the cleavage site, wherein the targeting endonuclease introduces a double-stranded break into the chromosomal sequence, and the double-stranded break is repaired by (i) a non-homologous end-joining repair
process such that an inactivating mutation is introduced into the chromosomal sequence, or (ii) a homology-directed repair process such that the sequence in the donor polynucleotide is integrated into the chromosomal sequence. The method further comprises culturing the cell such that the targeting endonuclease introduces a double-stranded break at the targeted site in the chromosomal sequence. Upon repair of the double-stranded break, the edited chromosomal sequence comprises a deletion of least nucleotide, an insertion of at least one nucleotide, substitution of at least one nucleotide, or combinations thereof.

The type of targeting endonuclease used in the method disclosed herein can and will vary. The targeting endonuclease may be a naturally-occurring protein or an engineered protein. In one embodiment, the targeting endonuclease may be a meganuclease. Meganucleases are endodeoxyribonucleases characterized by a large recognition site, i.e., the recognition site generally ranges from about 12 base pairs to about 40 base pairs. As a consequence of this requirement, the recognition site generally occurs only once in any given genome. Among meganucleases, the LAGLIDADG family of homing endonucleases has become a valuable tool for the study of genomes and genome engineering. Meganucleases can be targeted to specific chromosomal sequence by modifying their recognition sequence using techniques well known to those skilled in the art.

In another embodiment, the targeting endonuclease may be a transcription activator-like effector (TALE) nuclease. TALEs are transcription factors from the plant pathogen Xanthomonas that can be readily engineered to bind new DNA targets. TALEs or truncated versions thereof may be linked to the catalytic domain of endonucleases such as Fokl to create targeting endonuclease called TALE nucleases orTALENs.

In still another embodiment, the targeting endonuclease may be a site-specific nuclease. In particular, the site-specific nuclease may be a "rare-cutter" endonuclease whose recognition sequence occurs rarely in a genome. Preferably, the recognition sequence of the site-specific nuclease occurs only once in a genome. In an alternate further embodiment, the targeting nuclease may be an artificial targeted DNA double strand break inducing agent.

### (i) Zinc finger nuclease-mediated genomic editing

In a preferred embodiment, the targeting endonuclease may be a zinc finger nuclease (ZFN). Typically, a zinc finger nuclease comprises a DNA binding domain (i.e., zinc finger) and a cleavage domain (i.e., nuclease), both of which are described below.

*Zinc finger binding domain.* Zinc finger binding domains may be engineered to recognize and bind to any nucleic acid sequence of choice. See, for example, Beerli et al. (2002) Nat. Biotechnol. 20:135-141; Pabo et al. (2001) Ann. Rev. Biochem. 70:313-340; Isalan et al. (2001) Nat. Biotechnol. 19:656-660; Segal et al. (2001) Curr. Opin. Biotechnol. 12:632-637; Choo et al. (2000) Curr. Opin. Struct. Biol. 10:411-416; Zhang et al. (2000) J. Bioi. Chem. 275(43):33850-33860; Doyon et al. (2008) Nat. Biotechnol. 26:702-708; and Santiago et al. (2008) Proc. Natl. Acad. Sci. USA 105:5809-5814. An engineered zinc finger binding domain may have a novel binding specificity compared to a naturally-occurring zinc finger protein. Engineering methods include, but are not limited to, rational design and various types of selection. Rational design includes, for example, using databases comprising doublet, triplet, and/or quadruplet nucleotide sequences and individual zinc finger amino acid sequences, in which each doublet, triplet or quadruplet nucleotide sequence is associated with one or more amino acid sequences of zinc fingers which bind the particular triplet or quadruplet sequence. See, for example, U.S. Pat. Nos. 6,453,242 and 6,534,261. As an example, the algorithm of described in US patent 6,453,242 may be used to design a zinc finger binding domain to target a preselected sequence. Alternative methods, such as rational design using a nondegenerate recognition code table may also be used to design a zinc finger binding domain to target a specific sequence (Sera et al. (2002) Biochemistry 41 :7074-7081). Publically available web based tools for identifying potential target sites in DNA sequences and designing zinc
finger binding domains may be found at http://www.zincfingertools.org and http://bindr.gdcb.iastate.edu/ZiFiT/, respectively (Mandell et al. (2006) Nuc. Acid Res. 34:W516-W523; Sander et al. (2007) Nuc. Acid Res. 35:W599-W605).

A zinc finger binding domain may be designed to recognize and bind a DNA sequence ranging from about 3 nucleotides to about 21 nucleotides in length, or preferably from about 9 to about 18 nucleotides in length. In general, the zinc finger binding domains of the zinc finger nucleases disclosed herein comprise at least three zinc finger recognition regions (i.e., zinc fingers). In one embodiment, the zinc finger binding domain may comprise four zinc finger recognition regions. In another embodiment, the zinc finger binding domain may comprise five zinc finger recognition regions. In still another embodiment, the zinc finger binding domain may comprise six zinc finger recognition regions. A zinc finger binding domain may be designed to bind to any suitable target DNA sequence. See for example, U.S. Pat. Nos. 6,607,882; 6,534,261 and 6,453,242.

Exemplary methods of selecting a zinc finger recognition region may include phage display and two-hybrid systems, and are disclosed in U.S. Pat. Nos. 5,789,538; 5,925,523; 6,007,988; 6,013,453; 6,410,248; 6,140,466; 6,200,759; and 6,242,568; as well as WO 98/37186; WO 98/53057; WO 00/27878; WO 01/88197 and GB 2,338,237, each of which is incorporated by reference herein in its entirety. In addition, enhancement of binding specificity for zinc finger binding domains has been described, for example, in WO 02/077227.

Zinc finger binding domains and methods for design and construction of fusion proteins (and polynucleotides encoding same) are known to those of skill in the art and are described in detail in U.S. Patent Application Publication Nos. 20050064474 and 20060188987. Zinc finger recognition regions and/or multi-fingered zinc finger proteins may be linked together using suitable linker sequences, including for example, linkers of five or more amino acids in length. See U.S. Pat. Nos. 6,479,626; 6,903,185; and 7,153,949,
for non- limiting examples of linker sequences of six or more amino acids in length. The zinc finger binding domain described herein may include a combination of suitable linkers between the individual zinc fingers of the protein.

In some embodiments, the zinc finger nuclease may further comprise a nuclear localization signal or sequence (NLS). A NLS is an amino acid sequence which facilitates targeting the zinc finger nuclease protein into the nucleus to introduce a double stranded break at the target sequence in the chromosome. Nuclear localization signals are known in the art. See, for example, Makkerh et al. (1996) Current Biology 6:1025-1027.

*Cleavage domain.* A zinc finger nuclease also includes a cleavage domain. The cleavage domain portion of the zinc finger nuclease may be obtained from any endonuclease or exonuclease. Non-limiting examples of endonucleases from which a cleavage domain may be derived include, but are not limited to, restriction endonucleases and homing endonucleases. See, for example, 2002-2003 Catalog, New England Biolabs, Beverly, Mass.; and Belfort et al. (1997) Nucleic Acids Res. 25:3379-3388 or www.neb.com. Additional enzymes that cleave DNA are known (e.g., S1 Nuclease; mung bean nuclease; pancreatic DNase I; micrococcal nuclease; yeast HO endonuclease). See also Linn et al. (eds.) Nucleases, Cold Spring Harbor Laboratory Press, 1993. One or more of these enzymes (or functional fragments thereof) may be used as a source of cleavage domains.

A cleavage domain also may be derived from an enzyme or portion thereof, as described above, that requires dimerization for cleavage activity. Two zinc finger nucleases may be required for cleavage, as each nuclease comprises a monomer of the active enzyme dimer. Alternatively, a single zinc finger nuclease may comprise both monomers to create an active enzyme dimer. As used herein, an "active enzyme dimer" is an enzyme dimer capable of cleaving a nucleic acid molecule. The two cleavage monomers may be derived from the same endonuclease (orfunctional fragments thereof), or each monomer may be derived from a different endonuclease (or functional fragments thereof).

When two cleavage monomers are used to form an active enzyme dimer, the recognition sites for the two zinc finger nucleases are preferably disposed such that binding of the two zinc finger nucleases to their respective recognition sites places the cleavage monomers in a spatial orientation to each other that allows the cleavage monomers to form an active enzyme dimer, e.g., by dimerizing. As a result, the near edges of the recognition sites may be separated by about 5 to about 18 nucleotides. For instance, the near edges may be separated by about 5, 6, 7, 8, 9, 10, 11, 12, 13, 14, 15, 16, 17 or 18 nucleotides. It will however be understood that any integral number of nucleotides or nucleotide pairs may intervene between two recognition sites (e.g., from about 2 to about 50 nucleotide pairs or more). The near edges of the recognition sites of the zinc finger nucleases, such as for example those described in detail herein, may be separated by 6 nucleotides. In general, the site of cleavage lies between the recognition sites.

Restriction endonucleases (restriction enzymes) are present in many species and are capable of sequence-specific binding to DNA (at a recognition site), and cleaving DNA at or near the site of binding. Certain restriction enzymes (e.g., Type IIS) cleave DNA at sites removed from the recognition site and have separable binding and cleavage domains. For example, the Type IIS enzyme Fokl catalyzes double-stranded cleavage of DNA, at 9 nucleotides from its recognition site on one strand and 13 nucleotides from its recognition site on the other. See, for example, U.S. Pat. Nos. 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Bioi. Chem. 269:31, 978-31, 982. Thus, a zinc finger nuclease may comprise the cleavage domain from at least one Type IIS restriction enzyme and one or more zinc finger binding domains, which may or may not be engineered. Exemplary Type IIS restriction enzymes are described for example in International Publication WO 07/014,275. Additional restriction enzymes also contain separable binding and cleavage domains,
and these also are contemplated by the present disclosure. See, for example, Roberts et al. (2003) Nucleic Acids Res. 31:418-420.

An exemplary Type IIS restriction enzyme, whose cleavage domain is separable from the binding domain, is Fokl. This particular enzyme is active as a dimer (Bitinaite et al. (1998) Proc. Natl. Acad. Sci. USA 95: 10, 570-10, 575). Accordingly, for the purposes of the present disclosure, the portion of the Fokl enzyme used in a zinc finger nuclease is considered a cleavage monomer. Thus, for targeted double-stranded cleavage using a Fokl cleavage domain, two zinc finger nucleases, each comprising a Fokl cleavage monomer, may be used to reconstitute an active enzyme dimer. Alternatively, a single polypeptide molecule containing a zinc finger binding domain and two Fokl cleavage monomers may also be used.

In certain embodiments, the cleavage domain may comprise one or more engineered cleavage monomers that minimize or prevent homodimerization, as described, for example, in U.S. Patent Publication Nos. 20050064474, 20060188987, and 20080131962. By way of non-limiting example, amino acid residues at positions 446, 447, 479, 483, 484, 486, 487, 490, 491, 496, 498, 499, 500, 531, 534, 537, and 538 of Fokl are all targets for influencing dimerization of the Fokl cleavage half-domains. Exemplary engineered cleavage monomers of Fokl that form obligate heterodimers include a pair in which a first cleavage monomer includes mutations at amino acid residue positions 490 and 538 of Fokl and a second cleavage monomer that includes mutations at amino-acid residue positions 486 and 499.

Thus, in one embodiment, a mutation at amino acid position 490 replaces Glu (E) with Lys (K); a mutation at amino acid residue 538 replaces Iso (I) with Lys (K); a mutation at amino acid residue 486 replaces Gin (Q) with Glu (E); and a mutation at position 499 replaces Iso (I) with Lys (K). Specifically, the engineered cleavage monomers may be prepared by mutating positions 490 from E to K and 538 from I to K one cleavage monomer to produce an engineered cleavage monomer designated "E490K:I538K" and by mutating positions 486 from Q to E and 499 from I to L another cleavage monomer to produce an engineered cleavage monomer
designated "Q486E:1499L." The above described engineered cleavage monomers are obligate heterodimer mutants in which aberrant cleavage is minimized or abolished. Engineered cleavage monomers may be prepared using a suitable method, for example, by site-directed mutagenesis of wild-type cleavage monomers (Fokl) as described in U.S. Patent Publication No. 20050064474.

### (ii) Optional donor polynucleotide

The method for targeted genome editing may further comprise introducing into the cell at least one donor polynucleotide comprising a sequence to be integrated into the chromosomal sequence. For example, the sequence to be integrated into the chromosomal sequence (also called the sequence of interest) may comprise at least one nucleotide substitution. A donor polynucleotide comprises the sequence of interest, as well as a sequence that is substantially identical to a sequence upstream of the site of integration, and a sequence that is substantially identical to a sequence downstream of the site of integration. Thus, within the donor polynucleotide, the sequence of interest is flanked by the upstream and downstream sequences, wherein the upstream and downstream sequences share sequence similarity with either side of the site of integration in the chromosome.

Typically, the donor polynucleotide will be DNA. The DNA may be single-stranded or double-stranded. The donor polynucleotide may be a DNA plasmid, a bacterial artificial chromosome (BAC), a yeast artificial chromosome (YAC), a viral vector, a linear piece of DNA, a PCR fragment, a naked nucleic acid, or a nucleic acid complexed with a delivery vehicle such as a liposome or poloxamer.

The upstream and downstream sequences in the donor polynucleotide are selected to promote recombination between the chromosomal sequence of interest and the donor polynucleotide. The upstream sequence, as used herein, refers to a nucleic acid sequence that shares sequence similarity with the chromosomal sequence upstream of the targeted site of integration. Similarly, the downstream sequence refers to a nucleic acid sequence that shares sequence similarity with the chromosomal sequence downstream of the targeted site of integration. The upstream and downstream sequences in the donor polynucleotide may share about 75%, 76%, 77%, 78%, 79%, 80%, 81%, 82%, 83%, 84%, 85%, 86%, 87%, 88%, 89%, 90%, 91%, 92%, 93%, or 94% sequence identity with the targeted chromosomal sequence. In other embodiments, the upstream and downstream sequences in the donor polynucleotide may share about 95%, 96%, 97%, 98%, 99%, or 100% sequence identity with the targeted chromosomal sequence. In an exemplary embodiment, the upstream and downstream sequences in the donor polynucleotide may share about 99% or 100% sequence identity with the targeted chromosomal sequence.

An upstream or downstream sequence may comprise from about 20 bp to about 2500 bp. In one embodiment, an upstream or downstream sequence may comprise about 20, 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. A preferred upstream or downstream sequence may comprise about 200 bp to about 2000 bp, about 600 bp to about 1000 bp, or more particularly about 700 bp to about 1000 bp.

In some embodiments, the donor polynucleotide may further comprise a marker. Such a marker may make it easy to screen for targeted integrations. Non-limiting examples of suitable markers include restriction sites, fluorescent proteins, or selectable markers.

One of skill in the art would be able to construct a donor polynucleotide as described herein using well-known standard recombinant techniques (see, for example, Sambrook et al., 2001 and Ausubel et al., 1996).

### (iii) Delivery to the cell

The targeting endonuclease may be introduced into the cell as a nucleic acid that encodes the targeting endonuclease. The nucleic acid may be DNA or RNA. In embodiments in which the targeting endonuclease is encoded by mRNA, the mRNA may be 5' capped and/or 3' polyadenylated. In embodiments in which the targeting endonuclease is encoded by DNA, the DNA may be linear or circular. The DNA may be part of a vector, wherein the encoding DNA may be operably linked to a suitable promoter. Those skilled in the art are familiar with appropriate vectors, promoters, other control elements, and means of introducing the vector into the cell of interest.

The method comprises introducing the targeting endonuclease or a nucleic acid encoding the targeting endonuclease, and optionally, a donor polynucleotide into the cell. Suitable methods of introducing the desired molecules into the cell include microinjection, electroporation, sonoporation, biolistics, calcium phosphate-mediated transfection, cationic transfection, liposome transfection, dendrimer transfection, heat shock transfection, nucleofection transfection, magnetofection, lipofection, impalefection, optical transfection, proprietary agent-enhanced uptake of nucleic acids, and delivery via liposomes, immunoliposomes, virosomes, or artificial virions.

In embodiments in which both a nucleic acid encoding a targeting endonuclease and a donor polynucleotide are introduced into a cell, the ratio of donor polynucleotide to nucleic acid encoding a zinc finger nuclease may range from about 1:10 to about 10:1. In various embodiments, the ratio of donor polynucleotide to nucleic acid encoding a zinc finger nuclease may be about 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or 10:1. In one embodiment, the ratio may be about 1:1.

In embodiments in which more than one nucleic acid encoding a targeting endonuclease and, optionally, more than one donor polynucleotide are introduced into a cell, the nucleic acids may be introduced simultaneously or sequentially. For example, nucleic acids encoding the targeting endonucleases, each specific for a distinct recognition sequence, as well as the optional donor polynucleotides, may be introduced at the same time. Alternatively, each nucleic acid encoding a targeting endonuclease, as well as the optional donor polynucleotides may be introduced sequentially.

### (iv) Culturing the cell

The method of editing a chromosomal sequence with a targeting endonuclease further comprises culturing the cell to permit expression of the targeting endonuclease, if necessary, cleavage at the targeted site, and repair of the double-stranded break induced by the targeting endonuclease. Cells will generally be cultured using standard cell culture techniques as described, for example, in Santiago et al. (2008) PNAS 105:5809-5814; Moehle et al. (2007) PNAS 104:3055-3060; Urnov et al. (2005) Nature 435:646-651; and Lombardo et al (2007) Nat. Biotechnology 25:1298-1306. Those of skill in the art appreciate that methods for culturing cells are known in the art and can and will vary depending on the cell type. Routine optimization may be used, in all cases, to determine the best techniques for a particular cell type.

In embodiments in which the cell comprises a targeting endonuclease but no donor polynucleotide, the targeting endonuclease recognizes, binds, and cleaves the target sequence in the chromosomal sequence of interest. The double-stranded break introduced by the targeting endonuclease is repaired by an error-prone non-homologous end-joining DNA repair process. Consequently, a deletion or insertion of at least one nucleotide may be introduced in the chromosomal sequence. As a result of the deletion or insertion, the chromosomal sequence may be inactivated such that no functional transporter is made.

In embodiments in which the cell comprises a targeting endonuclease as well as a donor polynucleotide, the targeting endonuclease recognizes, binds, and cleaves the target sequence in the chromosome. The double stranded break introduced into the chromosomal sequence by the targeting endonuclease is repaired, via homologous recombination with the donor polynucleotide, such that the sequence in the donor polynucleotide may be exchanged with a portion of the chromosomal sequence. The donor polynucleotide may be physically integrated or, alternatively, the donor polynucleotide may be used as a template for repair of the break, resulting in the exchange of the sequence information in the donor polynucleotide with the sequence information in that portion of the chromosomal sequence. Thus, a portion of the endogenous chromosomal sequence may be converted to the sequence of the donor polynucleotide. The changed nucleotide(s) may be at or near the site of cleavage. Alternatively, the changed nucleotide(s) may be anywhere in the exchanged sequences. As a consequence of the exchange, however, the chromosomal sequence is modified, such that a modified version of the ADME/Tox associated protein may be made or, alternatively, the level of expression may be altered.

### (b) RNA Interference

Also disclosed herein the, expression of at least one ADME/Tox protein may be disrupted using RNA interference technology. That is, expression may be disrupted by introducing an RNA interference (RNAi) agent that inhibits expression of a target mRNA or transcript. The RNAi agent may lead to cleavage and degradation of the target mRNA or transcript. Alternatively, the RNAi agent may prevent or disrupt translation of the target mRNA into protein.

As an example, the RNAi agent may be a short interfering RNA (siRNA). In general, a siRNA comprises a double-stranded RNA molecule that ranges from about 15 to about 29 nucleotides in length. The siRNA may be about 16-18, 17-19,21-23, 24-27, or 27-29 nucleotides in length. The siRNA may be about 21 nucleotides in length. The siRNA may optionally further comprise one or two single-stranded overhangs, e.g., a 3' overhang on one or both ends. The siRNA may be formed from two RNA molecules that hybridize together or, alternatively, may be generated from a short hairpin RNA (shRNA) (see below). The two strands of the siRNA may be completely complementary, such that no mismatches or bulges exist in the duplex formed between the two sequences. Alternatively, the two strands of the siRNA may be substantially complementary, such that one or more mismatches and/or bulges may exist in the duplex formed between the two sequences. One or both of the 5' ends of the siRNA may have a phosphate group. Alternatively, one or both of the 5' ends may lack a phosphate group. One or both of the 3' ends of the siRNA may have a hydroxyl group. Alternatively one or both of the 5' ends may lack a hydroxyl group.

One strand of the siRNA, which is referred to as the "antisense strand" or "guide strand," includes a portion that hybridizes with the target transcript. The antisense strand of the siRNA may be completely complementary with a region of the target transcript, i.e., it hybridizes to the target transcript without a single mismatch or bulge over a target region between about 15 and about 29 nucleotides in length, preferably at least 16 nucleotides in length, and more preferably about 18-20 nucleotides in length. The antisense strand may be substantially complementary to the target region, i.e., one or more mismatches and/or bulges may exist in the duplex formed by the antisense strand and the target transcript. Typically, siRNAs are targeted to exonic sequences of the target transcript. Those of skill in the art are familiar with programs, algorithms, and/or commercial services that design siRNAs for target transcripts. An exemplary example is the Rosetta siRNA Design Algorithm (Rosetta Inpharmatics, North Seattle, WA) and MISSION® siRNA (Sigma-Aldrich, St. Louis, MO). The siRNA may be enzymatically synthesized in vitro using methods well known to those of skill in the art. Alternatively, the siRNA may be chemically synthesized using oligonucleotide synthesis techniques that are well known in the art.

Disclosed herein, the RNAi agent may be a short hairpin RNA (shRNA). In general, a shRNA is an RNA molecule comprising at least two complementary portions that are hybridized or are capable of hybridizing to form a double-stranded structure sufficiently long to mediate RNA interference (as described above), and at least one single-stranded portion that forms a loop connecting the regions of the shRNA that form the duplex. The structure may also be called a stem- loop structure, with the stem being the duplex portion. The duplex portion of the structure may be completely complementary, such that no mismatches or bulges exist in the duplex region of the shRNA. Alternatively, the duplex portion of the structure may be substantially complementary, such that one or more mismatches and/or bulges may exist in the duplex portion of the shRNA. The
loop of the structure may be from about 1 to about 20 nucleotides in length, preferably from about 4 to about 10 about nucleotides in length, and more preferably from about 6 to about 9 nucleotides in length. The loop may be located at either the 5' or 3' end of the region that is complementary to the target transcript (i.e., the antisense portion of the shRNA).

The shRNA may further comprise an overhang on the 5' or 3' end. The optional overhang may be from about 1 to about 20 nucleotides in length, and more preferably from about 2 to about 15 nucleotides in length. The overhang may comprise one or more U residues, e.g., between about 1 and about 5 U residues. The 5' end of the shRNA may have a phosphate group, while in other embodiments it may not. The 3' end of the shRNA may have a hydroxyl group, while in other embodiments it may not. In general, shRNAs are processed into siRNAs by the conserved cellular RNAi machinery. Thus, shRNAs are precursors of siRNAs and are similarly capable of inhibiting expression of a target transcript that is complementary of a portion of the shRNA (i.e., the antisense portion of the shRNA). Those of skill in the art are familiar with the available resources (as detailed above) for the design and synthesis of shRNAs. An exemplary example is MISSION® shRNAs (Sigma-Aldrich).

The RNAi agent may be an RNAi expression vector. Typically, an RNAi expression vector may be used for intracellular (in vivo) synthesis of RNAi agents, such as siRNAs or shRNAs. Two separate, complementary siRNA strands may be transcribed using a single vector containing two promoters, each of which directs transcription of a single siRNA strand (i.e., each promoter is operably linked to a template for the siRNA so that transcription may occur). The two promoters may be in the same orientation, in which case each is operably linked to a template for one of the complementary siRNA strands. Alternatively, the two promoters may be in opposite orientations, flanking a single template so that transcription for the promoters results in synthesis of two complementary siRNA strands. The RNAi expression vector may contain a promoter that drives transcription of a single RNA molecule comprising two complementary regions, such that the transcript forms a shRNA.

Those of skill in the art will appreciate that it is preferable for siRNA and shRNA agents to be produced in vivo via the transcription of more than one transcription unit. Generally speaking, the promoters utilized to direct in vivo expression of the one or more siRNA or shRNA transcription units may be promoters for RNA polymerase III (Pol III). Certain Pol III promoters, such as U6 or H1 promoters, do not require cis-acting regulatory elements within the transcribed region, and thus, are preferred in certain embodiments. Promoters for Pol II may be used to drive expression of the one or more siRNA or shRNA transcription units. Tissue-specific, cell-specific, or inducible Pol II promoters may be used.

A construct that provides a template for the synthesis of siRNA or shRNA may be produced using standard recombinant DNA methods and inserted into any of a wide variety of different vectors suitable for expression in eukaryotic cells. Guidance may be found in Current Protocols in Molecular Biology (Ausubel et al., John Wiley & Sons, New York, 2003) or Molecular Cloning: A Laboratory Manual (Sambrook & Russell, Cold Spring Harbor Press, Cold Spring Harbor, NY, 3rd edition, 2001). Those of skill in the art also appreciate that vectors may comprise additional regulatory sequences (e.g., termination sequence, translational control sequence, etc.), as well selectable marker sequences. DNA plasmids are known in the art, including those based on pBR322, PUC, and so forth. Since many expression vectors already contain a suitable promoter or promoters, it may be only necessary to insert the nucleic acid sequence that encodes the RNAi agent of interest at an appropriate location with respect to the promoter(s). Viral vectors may also be used to provide intracellular expression of RNAi agents. Suitable viral vectors include retroviral vectors, lentiviral vectors, adenoviral vectors, adeno-associated virus vectors, herpes virus vectors, and so forth. In preferred embodiment, the RNAi expression vector is a shRNA lentiviral- based vector or lentiviral particle, such as that provided in MISSION® TRC shRNA products (Sigma-Aldrich).

The RNAi agents or RNAi expression vectors may be introduced into the cell using methods well known to those of skill in the art. Guidance may be found in Ausubel et al., supra or Sambrook & Russell, supra, for example. In some embodiments, the RNAi expression vector, e.g., a viral vector, may be stably integrated into the genome of the cell, such that cell stress related protein expression is disrupted over subsequent cell generations.

### (c) Homologous Recombination

Also disclosed herein, homologous recombination techniques may be used to disrupt expression of a ADME/Tox protein/gene at the level of the genomic DNA. Accordingly, these techniques may be used to delete a nucleic acid sequence, delete a portion of a nucleic acid sequence, or introduce point mutations in the nucleic acid sequence, such that no functional biomarker may be made. The nucleic acid sequence may be targeted by homologous recombination using the techniques of Capecchi (Cell 22:4779-488, 1980) and Smithies (Proc Natl Acad Sci USA 91 :3612-3615, 1994). Alternatively, the nucleic acid sequence may be targeted using a Cre-/oxP site-specific recombination system, a Flp-FRT site-specific recombination system, or variants thereof. Such recombination systems are
commercially available, and additional guidance may be found in Ausubel et al., supra.

### (d) Measuring Disrupted Expression

The methods described above for disrupting expression generally will lead to altered (i.e., decreased or increased) or no expression of targeted gene(s) and, consequently, altered (i.e., decreased or increased) or no activity of the protein encoded by the gene. A wide variety of methods known in the art may be used to measure mRNA levels, protein levels, or enzyme activity. Non-limiting examples of RNA detection methods include reverse transcriptase PCR, reverse transcriptase quantitative PCR, nucleic acid microarrays, hybridization-based methods, branched DNA detection technologies, Northern blotting, and nuclease protection assays. Non- limiting examples of protein detection methods include Western blotting, ELISA assays,
and other immunoassays. Depending upon the specificity of the probes used in these detection assays, each targeted ADME/Tox protein may be detected singly or all targeted ADME/Tox proteins may be detected simultaneously. Regardless of the specificity, however, a decrease in the level of expression may be determined by comparing the levels (of mRNA and/or protein) in a cell comprising disrupted expression of the at least one ADME/Tox protein to those in a cell in which expression of the ADME/Tox protein is not disrupted.

The DNA sequence of the ADME/Tox gene may be modified such that expression is substantially eliminated. For example, some or all of the DNA sequence may be deleted (or altered) such that no functional product is made. Such cells may be termed knockout cells. Expression of the ADME/Tox protein may be disrupted such that reduced levels of mRNA and/or protein are made. Such cells may be termed knock-down cells. For example, the levels of mRNA and/or protein of the targeted ADME/Tox gene may be reduced by at least about 5%, 10%, 20%, 30%, 40%, 50%, 60%, 70%, 80%, 90%, 95%, or 99% relative to those of cells in which expression is not disrupted. The DNA sequence of the ADME/Tox gene may be modified (e.g., via a SNP) such that a modified protein product is made. The modified protein product may have altered substrate specificities, altered binding properties, altered stability, altered kinetics, and the like.

### (III) Method for Assessing the Effect of an Agent in a Cell

Also provided herein is a method for assessing the effect of an agent, wherein the method comprises contacting a cell having disrupted expression of an endogenous genomic sequence ABCG2, wherein expression is disrupted by targeting endonuclease mediated genome editing with the agent. The method further comprises comparing results of a selected parameter to results obtained from contacting a comparable wild-type cell with the same agent.

For example, the agent may be a pharmaceutically
active ingredient, a therapeutic agent, a chemotherapeutic agent, an antibiotic, an antimicrobial agent, an analgesic, an anti-inflammatory agent, a small molecule, a biologic, a food additive, a pesticide, a herbicide, a toxin, an industrial chemical, or an environmental chemical.

Non-limiting examples of suitable parameters to measure include permeability, efflux ratio, enzyme activity, enzyme kinetics, substrate binding, transcriptional activity, protein translocation, redox signaling, and so forth.

In general, the effect(s) of the agent may be assessed within the context of absorption, distribution, metabolism, excretion, and/or toxicology of the candidate agent. For example, genetic modification such as knockout of BCRPI and any one or more of MDR1, MRP1, and MRP2 may be used to characterize a candidate drug's ADME profile in a cell line cell model for disease, for initial identification of a potential therapeutic compound, and for lead optimization.

For example, a drug candidate may be evaluated with respect to multi-drug resistance, which refers to the ability of cells to develop resistance to a broad range of drugs that may be structurally and/or functionally unrelated. "Multidrug resistance" also encompasses cross-resistance between drugs. As detailed above, several transporters are involved in the development of multidrug resistance, which occurs when the drug is transported out of the cell. For example, multidrug resistance of certain tumors to chemotherapy agents involves ABC transporter proteins. Such transport may be mediated for example by any one or more ABC transporters, including MDR1 a, MDR1 b, BCRP, MRP1, or MRP2, or homologs thereof. Thus, genetic modification such as a knockout of an ABC transporter protein as described herein may be used, for example, to reduce the activity of an efflux ABC transporter protein or its homolog(s) to promote delivery of a drug through membranes which otherwise would exclude the drug. In particular, efflux inhibitors can be used to aid transport of a drug through the blood-brain barrier, or through the blood-testis barrier.

Also disclosed is a method for assessing the therapeutic efficacy of a potential gene therapy strategy. That is, a chromosomal sequence encoding an ADME/Tox protein may be modified such that a disorder or symptom related to mutation of an ADME/Tox gene are reduced or eliminated. In particular, the method comprises editing a chromosomal sequence encoding a ADME/Tox protein such that an altered protein product is produced. The genetically modified cell may be tested by exposure to various test conditions and cellular, and/or molecular responses measured and compared to those of a wild-type cell exposed to the same test conditions. Consequently, the therapeutic potential of the ADME/Tox gene therapy regime may be assessed.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

When introducing elements of the present disclosure or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

The term "disrupted expression" refers to the altered expression of gene/protein of interest. For example, expression of the gene product may be eliminated, reduced, or increased relative to the normal (non-disrupted) situation Alternatively, the expressed gene product may be altered or modified relative to the normal (non-disrupted) gene product.

As used herein, the term "endogenous" refers to a chromosomal sequence that is native to the cell.

The terms "editing," "genome editing," or "chromosomal editing" refer to a process by which a specific chromosomal sequence is changed. The edited chromosomal sequence may comprise an insertion of at least one nucleotide, a deletion of at least one nucleotide, and/or a substitution of at least one nucleotide.

A "gene," as used herein, refers to a DNA region (including exons and introns) encoding a gene product, as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites, and locus control regions.

As used herein, the terms "inactivated sequence," "inactivated chromosomal sequence," or "inactivated genomic sequence" refers to a genomic or chromosomal sequence that has been modified by a deletion of at least one nucleotide, an insertion of at least one nucleotide, and/or a substitution of at least one nucleotide such that expression of the encoded product is substantially eliminated.

The terms "nucleic acid" and "polynucleotide" refer to a deoxyribonucleotide or ribonucleotide polymer, in linear or circular conformation. For the purposes of the present disclosure, these terms are not to be construed as limiting with respect to the length of a polymer. The terms can encompass known analogs of natural nucleotides, as well as nucleotides that are modified in the base, sugar and/or phosphate moieties (e.g., phosphorothioate backbones). In general, an analog of a particular nucleotide has the same base-pairing specificity; i.e., an analog of A will base-pair with T.

The terms "polypeptide" and "protein" are used interchangeably to refer to a polymer of amino acid residues.

As used herein, the terms "target site" or "target sequence" refer to a nucleic acid sequence that defines a portion of a chromosomal sequence to be edited and to which a targeting endonuclease is engineered to recognize and bind, provided sufficient conditions for binding exist.

The terms "upstream" and "downstream" refer to locations in a nucleic acid sequence relative to a fixed position. Upstream refers to the region that is 5' (i.e., near the 5' end of the strand) to the position and downstream refers to the region that is 3' (i.e., near the 3' end of the strand) to the position.

Techniques for determining nucleic acid and amino acid sequence identity are known in the art. Typically, such techniques include determining the nucleotide sequence of the mRNA for a gene and/or determining the amino acid sequence encoded thereby, and comparing these sequences to a second nucleotide or amino acid sequence. Genomic sequences can also be determined and compared in this fashion. In general, identity refers to an exact nucleotide-to-nucleotide or amino acid-to-amino acid correspondence of two polynucleotides or polypeptide sequences, respectively. Two or more sequences (polynucleotide or amino acid) can be compared by determining their percent identity. The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman, Advances in Applied Mathematics 2:482-489 (1981). This algorithm can be applied to amino acid sequences by using the scoring matrix developed by Dayhoff, Atlas of Protein Sequences and Structure, M. O. Dayhoff ed., 5 suppl. 3:353-358, National Biomedical Research Foundation, Washington, D.C., USA, and normalized by Gribskov, Nucl. Acids Res. 14(6):6745-6763 (1986). An exemplary implementation of this algorithm to determine percent identity of a sequence is provided by the Genetics Computer Group (Madison, Wis.) in the "BestFit" utility application. Other suitable programs for calculating the percent identity or similarity between sequences are generally known in the art, for example, another alignment program is BLAST, used with default parameters. For example, BLASTN and BLASTP can be used using the following default parameters: genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+Swiss protein+Spupdate+PIR. Details of these programs can be found on the GenBank website. With respect to sequences described herein, the range of desired degrees of sequence identity is approximately 80% to 100% and any integer value therebetween. Typically the percent identities between sequences are at least 70-75%, preferably 80-82%, more preferably 85-90%, even more preferably 92%, still more preferably 95%, and most preferably 98% sequence identity.

As various changes could be made in the above cells, kits, and methods without departing from the scope of the invention, it is intended that all matter contained in the above description and in the examples given below, shall be interpreted as illustrative and not in a limiting sense.

### EXAMPLES

The following examples illustrate certain aspects of the invention.

### Example 1: BCRP Transporter Knockout and Validation in C2BBe1 Cells

The wild-type BCRP gene was knocked out in C2BBe1 cells using zinc finger nuclease technology to generate a stable cell line. Cells were sorted and single cell cloned. Cells were analyzed for the desired mutations and positive clones were chosen for further expansion and analysis. Quantitative PCR was used to evaluate mRNA expression of the cell and compared to wild type cell. Whole cell lysates prepared from these clones were subjected to immunoblotting for analysis of BCRP protein expression level. BCRP knockout cells showed greatly reduced mRNA and protein expression. A single BCRP knockout cell clone was selected for functional characterization of efflux activity using several known transporter-selective substrates (e.g. estrone 3-sulfate, digoxin and CDCFDA).

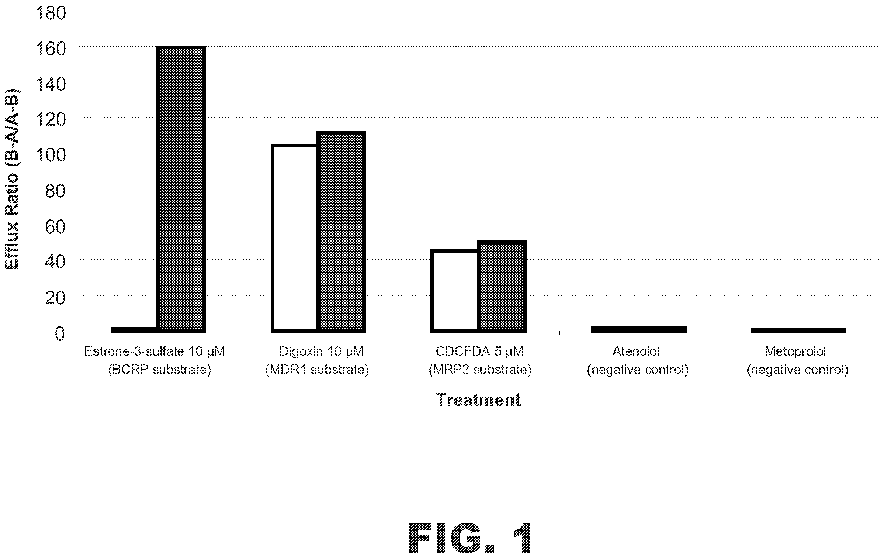

Both wild type cells (parental C2BBe1 cells) and BCRP knockout cells were seeded onto 24-well transwell plates and cultured for 21 days, following a standard protocol for intestinal efflux transporter assays previously developed using Caco-2 cells. The permeability of test compounds in both the apical to basal (A to B) and basal to apical (B to A) directions was measured following a two hour incubation and the efflux values calculated. The results are shown in FIG. 1. The efflux ratio of the known BCRP substrate, estrone 3-sulfate, was greatly reduced in the knockout cells compared to the parental cells, while the efflux ratio of the two other substrates was unaffected. Digoxin is a known MDR1 substrate, while CDCFDA is selective for the MRP2 transporter. Two additional compounds were used as controls, atenolol and metoprolol, which are known to not be substrates for either of these transporters.

### Example 2: Single and Double Efflux Transporter Knockouts in C2BBe1 Cells

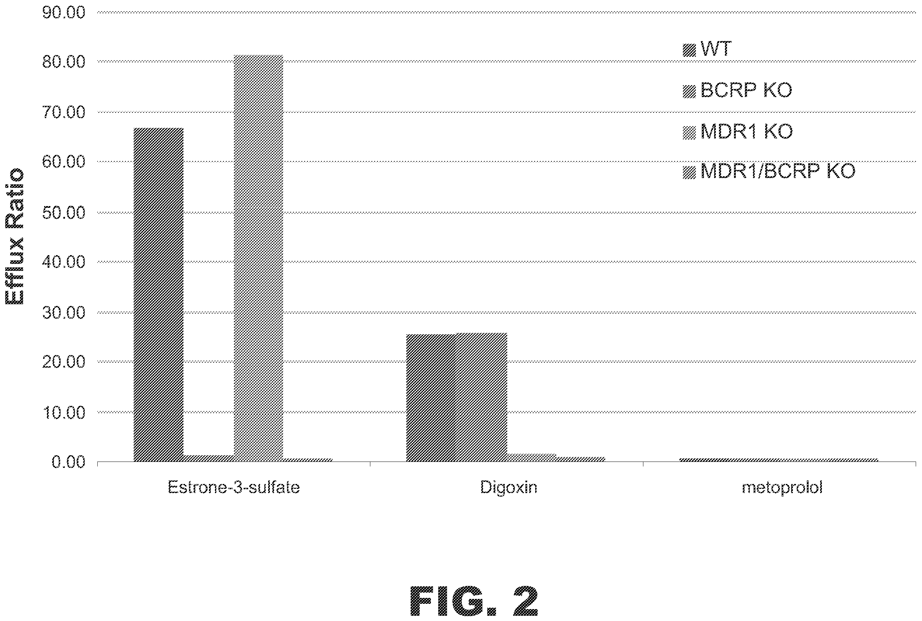

The BCRP and MDR1 transporter genes were individually knocked out and an MDR1/BCRP double knockout was also generated in C2BBe1 cells using zinc finger nuclease technology. Cells were analyzed and expanded as described above and then tested for functional activity in the standard transwell assay. Two substrates were utilized - estrone 3-sulfate as a selective substrate for BCRP and digoxin as a selective substrate for MDR1. Metoprolol was used as a control. As shown in FIG. **2****,** 4 cell lines were assessed, including the parental cell line. With estrone 3-sulfate, a high efflux ratio was seen in the parental line and in the MDR1 knockout, while the efflux was inhibited in both the BCRP single knockout and the MDR1/BCRP double knockout. For digoxin, the efflux ratio was high in the parental cell line and the BCRP individual knockout cells, but was inhibited in both the MDR1 knockout and the MDR1/BCRP double knockout, as expected.

## Claims

1. A genetically modified human cell line comprising disrupted expression of an endogenous genomic sequence encoding a protein involved in a drug absorption, distribution, metabolism and excretion and/or toxicology process, wherein the endogenous genomic sequence is ABCG2 and wherein expression is disrupted by targeting endonuclease mediated genome editing.

2. A method for assessing the effect of an agent, the method comprising:
a) contacting a human cell line comprising disrupted expression of an endogenous genomic sequence ABCG2 wherein expression is disrupted by targeting endonuclease mediated genome editing with the agent; and
b) comparing results of a selected parameter to results obtained from contacting a comparable wild-type cell with the same agent.

3. The method of claim 2, wherein the agent is chosen from a pharmaceutically active ingredient, a therapeutic agent, a chemotherapeutic agent, an antibiotic, an antimicrobial agent, an analgesic, an anti-inflammatory agent, a small molecule, a biologic, a food additive, a pesticide, a herbicide, a toxin, an industrial chemical or an environmental chemical.

4. The human cell line of claim 1 or method of claim 2, further comprising expression of at least one reporter protein.

5. The human cell line of claim 1 or method of claim 2, wherein expression is disrupted such that the cell produces no functional protein, a reduced level of the protein, an increased level of the protein, or an altered version of the protein.

6. The human cell line of claim 1 or method of claim 2, wherein a chromosomal sequence encoding the protein is modified by a deletion of at least one nucleotide, an insertion of at least one nucleotide, a substitution of at least one nucleotide, or a combination thereof.

7. The human cell line or method of claim 6, wherein the cell is heterozygous, homozygous, or hemizygous for the modified chromosomal sequence.

8. The human cell line of claim 1 or method of claim 2, wherein the cell comprises an inactivated ABCG2 chromosomal sequence such that it produces no BCRP protein and the cell comprises an inactivated ABBC1 chromosomal sequence such that it produces no MDR1 protein.

9. The human cell line or method of claim 8, further comprising expression of at least one reporter protein.

10. The human cell line or method of either claim 8 or claim 9, further comprising disrupted expression of at least one additional protein chosen from a membrane transporter, a drug metabolizing enzyme, a xenobiotic sensor, a cellular stress response pathway protein, and combinations thereof.

11. The human cell line or method of either claim 1 or claim 2 wherein expression is disrupted by targeting endonuclease mediated genome editing, and the targeting endonuclease is a zinc finger nuclease.

## Patentansprüche

1. Eine genetisch modifizierte menschliche Zelllinie, beinhaltend eine gestörte Expression einer endogenen genomischen Sequenz, welche ein Protein codiert, das an einem Prozess der Absorption, der Verteilung, des Metabolismus und der Ausscheidung und/oder der Toxikologie eines Arzneimittels beteiligt ist, wobei die endogene genomische Sequenz ABCG2 ist und wobei die Expression durch eine durch eine zielspezifische Endonuklease vermittelte Genomeditierung gestört ist.

2. Ein Verfahren zum Beurteilen der Wirkung eines Wirkstoffs, wobei das Verfahren Folgendes beinhaltet:
a) In-Kontakt-Bringen einer menschlichen Zelllinie, beinhaltend eine gestörte Expression einer endogenen genomischen Sequenz ABCG2, wobei die Expression durch eine durch eine zielspezifische Endonuklease vermittelte Genomeditierung gestört ist, mit dem Wirkstoff; und
b) Vergleichen der Ergebnisse eines ausgewählten Parameters mit Ergebnissen, die aus dem In-Kontakt-Bringen einer vergleichbaren Wildtypzelle mit dem gleichen Wirkstoff erhalten werden.

3. Verfahren gemäß Anspruch 2, wobei der Wirkstoff aus einem arzneilich wirksamen Bestandteil, einem therapeutischen Wirkstoff, einem chemotherapeutischen Wirkstoff, einem Antibiotikum, einem antimikrobiellen Wirkstoff, einem Analgetikum, einem entzündungshemmenden Wirkstoff, einem kleinen Molekül, einem Biologikum, einem Lebensmittelzusatzstoff, einem Pestizid, einem Herbizid, einem Toxin, einer Industriechemikalie oder einer Umweltchemikalie ausgewählt ist.

4. Menschliche Zelllinie gemäß Anspruch 1 oder Verfahren gemäß Anspruch 2, ferner beinhaltend die Expression mindestens eines Reporterproteins.

5. Menschliche Zelllinie gemäß Anspruch 1 oder Verfahren gemäß Anspruch 2, wobei die Expression so gestört ist, dass die Zelle kein funktionelles Protein, ein reduziertes Niveau des Proteins, ein erhöhtes Niveau des Proteins oder eine geänderte Version des Proteins herstellt.

6. Menschliche Zelllinie gemäß Anspruch 1 oder Verfahren gemäß Anspruch 2, wobei eine das Protein codierende chromosomale Sequenz durch eine Deletion mindestens eines Nukleotids, eine Insertion mindestens eines Nukleotids, eine Substitution mindestens eines Nukleotids oder eine Kombination davon modifiziert ist.

7. Menschliche Zelllinie oder Verfahren gemäß Anspruch 6, wobei die Zelle in Bezug auf die modifizierte chromosomale Sequenz heterozygot, homozygot oder hemizygot ist.

8. Menschliche Zelllinie gemäß Anspruch 1 oder Verfahren gemäß Anspruch 2, wobei die Zelle eine deaktivierte chromosomale ABCG2-Sequenz beinhaltet, so dass sie kein BCRP-Protein herstellt, und die Zelle eine deaktivierte chromosomale ABBC1-Sequenz beinhaltet, so dass sie kein MDR1-Protein herstellt.

9. Menschliche Zelllinie oder Verfahren gemäß Anspruch 8, ferner beinhaltend die Expression mindestens eines Reporterproteins.

10. Menschliche Zelllinie oder Verfahren gemäß entweder Anspruch 8 oder Anspruch 9, ferner beinhaltend eine gestörte Expression von mindestens einem zusätzlichen Protein, das aus einem Membrantransporter, einem Arzneimittel metabolisierenden Enzym, einem Xenobiotika-Sensor, einem Protein des zellulären Stressantwortwegs und Kombinationen davon ausgewählt ist.

11. Menschliche Zelllinie oder Verfahren gemäß entweder Anspruch 1 oder Anspruch 2, wobei die Expression durch eine durch eine zielspezifische Endonuklease vermittelte Genomeditierung gestört ist und die zielspezifische Endonuklease eine Zinkfingernuklease ist.

## Revendications

1. Une lignée de cellules humaines génétiquement modifiées comprenant l'expression perturbée d'une séquence génomique endogène codant pour une protéine impliquée dans un processus d'absorption, de distribution, de métabolisme et d'excrétion d'un médicament et/ou un processus de toxicologie, dans laquelle la séquence génomique endogène est ABCG2 et dans laquelle l'expression est perturbée par édition du génome médiée par une endonucléase ciblante.

2. Une méthode pour l'évaluation de l'effet d'un agent, la méthode comprenant :
a) la mise en contact d'une lignée de cellules humaines comprenant l'expression perturbée d'une séquence génomique endogène ABCG2 dans laquelle l'expression est perturbée par édition du génome médiée par une endonucléase ciblante avec l'agent ; et
b) la comparaison de résultats d'un paramètre sélectionné à des résultats obtenus à partir de la mise en contact d'une cellule de type sauvage comparable avec le même agent.

3. La méthode de la revendication 2, dans laquelle l'agent est choisi parmi un ingrédient pharmaceutiquement actif, un agent thérapeutique, un agent chimiothérapeutique, un antibiotique, un agent antimicrobien, un analgésique, un agent anti-inflammatoire, une petite molécule, un produit biologique, un additif alimentaire, un pesticide, un herbicide, une toxine, un produit chimique industriel ou un produit chimique environnemental.

4. La lignée de cellules humaines de la revendication 1 ou la méthode de la revendication 2, comprenant en outre l'expression d'au moins une protéine rapporteuse.

5. La lignée de cellules humaines de la revendication 1 ou la méthode de la revendication 2, dans laquelle l'expression est perturbée de manière que la cellule ne produise aucune protéine fonctionnelle, la cellule produise un niveau réduit de la protéine, un niveau accru de la protéine, ou une version changée de la protéine.

6. La lignée de cellules humaines de la revendication 1 ou la méthode de la revendication 2, dans laquelle une séquence chromosomique codant pour la protéine est modifiée par une délétion d'au moins un nucléotide, une insertion d'au moins un nucléotide, une substitution d'au moins un nucléotide, ou une combinaison de celles-ci.

7. La lignée de cellules humaines ou la méthode de la revendication 6, dans laquelle la cellule est hétérozygote, homozygote, ou hémizygote pour la séquence chromosomique modifiée.

8. La lignée de cellules humaines de la revendication 1 ou la méthode de la revendication 2, dans laquelle la cellule comprend une séquence chromosomique ABCG2 inactivée de manière qu'elle ne produise aucune protéine BCRP et la cellule comprend une séquence chromosomique ABBC1 inactivée de manière qu'elle ne produise aucune protéine MDR1.

9. La lignée de cellules humaines ou la méthode de la revendication 8, comprenant en outre l'expression d'au moins une protéine rapporteuse.

10. La lignée de cellules humaines ou la méthode de l'une ou l'autre de la revendication 8 ou de la revendication 9, comprenant en outre l'expression perturbée d'au moins une autre protéine choisie parmi un transporteur membranaire, une enzyme métabolisant un médicament, un capteur de xénobiotiques, une protéine d'une voie de réponse à un stress cellulaire, et des combinaisons de ceux-ci.

11. La lignée de cellules humaines ou la méthode de l'une ou l'autre de la revendication 1 ou de la revendication 2 dans laquelle l'expression est perturbée par édition du génome médiée par une endonucléase ciblante, et l'endonucléase ciblante est une nucléase à doigts de zinc.
